(19) Europäisches Patentamt
European Patent Office
Office européen des brevets

(11) EP 0 615 445 B1

(12) EUROPÄISCHE PATENTSCHRIFT

(45) Veröffentlichungstag und Bekanntmachung des Hinweises auf die Patenterteilung: **15.05.1996 Patentblatt 1996/20**

(21) Anmeldenummer: **92924547.0**

(22) Anmeldetag: **04.12.1992**

(51) Int. Cl.$^6$: **A61K 9/51**, A61K 9/10, A61K 47/42

(86) Internationale Anmeldenummer: **PCT/DE92/01010**

(87) Internationale Veröffentlichungsnummer: **WO 93/10768 (10.06.1993 Gazette 1993/14)**

# (54) PHARMAZEUTISCH APPLIZIERBARES NANOSOL UND VERFAHREN ZU SEINER HERSTELLUNG

PHARMACEUTICALLY APPLICABLE NANOSOL AND PROCESS FOR PREPARING THE SAME

NANOSOL UTILISABLE EN PHARMACIE ET SON PROCEDE DE PREPARATION

(84) Benannte Vertragsstaaten: **AT BE CH DE DK ES FR GB GR IT LI NL PT SE**

(30) Priorität: **05.12.1991 DE 4140195**
**05.12.1991 DE 4140177**
**05.12.1991 DE 4140178**

(43) Veröffentlichungstag der Anmeldung: **21.09.1994 Patentblatt 1994/38**

(73) Patentinhaber: **ALFATEC-PHARMA GmbH**
**D-69120 Heidelberg (DE)**

(72) Erfinder:
- **WUNDERLICH, Jens-Christian**
  **D-6900 Heidelberg (DE)**
- **SCHICK, Ursula**
  **D-6908 Wiesloch (DE)**
- **WERRY, Jürgen**
  **D-6700 Ludwigshafen (DE)**
- **FREIDENREICH, Jürgen**
  **D-6905 Schriesheim (DE)**

(74) Vertreter: **Fürniss, Peter, Dr.**
**Kuhnen, Wacker & Partner**
**Alois-Steinecker-Strasse 22**
**D-85354 Freising (DE)**

(56) Entgegenhaltungen:
**EP-A- 0 275 796** **EP-A- 0 282 020**
**EP-A- 0 349 428** **AU-A- 495 261**
**FR-A- 1 259 081** **FR-A- 2 608 427**

- **"Flüssige Arzneiformen schwerlöslicher Arzneistoffe", Seminar der APV in Fulda 23-25/1/89, Seiten 70-73,**
- **"Encapsin HPB, A real solution for drug delivery problems", Informationsbroschüre der Fa. Janssen**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 615 445 B1

Printed by Rank Xerox (UK) Business Services
2.11.7/3.4

## Beschreibung

Die vorliegende Erfindung betrifft ein Verfahren zur Herstellung eines kolloid-dispersen Systems von in Wasser schwerlöslichen anorganischen und/oder organischen Verbindungen gemäß Patentanspruch 1 bzw. von in Wasser schwer löslichen Arzneistoffen nach Anspruch 4. Die Erfindung betrifft weiterhin ein Nanosol von in Wasser schwer löslichen anorganischen und/oder organischen Verbindungen oder Gemischen von anorganischen und/oder organischen Verbindungen mit Gelatine gemäß Anspruch 25. Sie betrifft weiterhin ein pharmazeutisch applizierbares Nanosol, d. h. ein stabiles kolloid-disperses System von in Wasser schwerlöslichen Arzneistoffen mit Gelatine gemäß Anspruch 29. Weiterhin betrifft sie ein Akut-Arzneimittel zur Behandlung von rheumatischen und/oder entzündlichen Erkrankungen, das ein 3-Indolylessigsäurederivat enthält. Schließlich betrifft sie ein Akut-Arzneimittel zur Behandlung von Diabetes, das Glibenclamid enthält.

Die Schwierigkeit, Arzneistoffe mit problematischer Bioverfügbarkeit in eine befriedigende pharmazeutisch applizierbare Form zu bringen, ist allgemein bekannt. Etwa 30 % aller Wirkstoffe in Arzneimitteln fallen unter diese Gruppe. Für sie muß i. a. eine schnelle Freigabe des Wirkstoffs aus seiner Zubereitung nach der Applikation, d. h. eine schnelle Überführung in die gelöste, resorptionsfähige Form, gefordert werden, um einen akzeptablen Therapieerfolg zu erzielen. Setzt man voraus, daß der Resorptionsprozeß in vivo nicht der geschwindigkeitsbestimmende Schritt ist, lassen sich alle technologischen Verfahren zur Arzneistoff-Freigabeverbesserung auf die Beeinflussung zweier Parameter in der sogenannten Noyes-Whitney-Gleichung zurückführen:

$$\frac{dc}{dt} = \frac{D \,.\, A}{d \,.\, V}\,(c_s - c_t),$$

wobei

| | |
|---|---|
| $\frac{dc}{dt}$ | : in Lösung gehende Feststoffmenge pro Zeit = Lösungsgeschwindigkeit, |
| D | : Diffusionskoeffizient des betreffenden Stoffmoleküls |
| A | : effektive Feststoff- bzw. Kristalloberfläche, die dem Lösemittel zugänglich ist (benetzbare Oberfläche), |
| d | : Dicke der Diffusionsschicht |
| V | : Lösungsmittelvolumen, |
| $c_s$ | : Sättigungslöslichkeit des betreffenden Stoffs und |
| $c_t$ | : in Lösung herrschende Konzentration des betreffenden Stoffs zur Zeit t |

bedeuten. Diese Gleichung gibt einen mathematischen Ausdruck für die Lösegeschwindigkeit von Substanzen allgemein (hier Arzneistoffen) an. Dabei sind die für den Pharmazeuten änderbaren Zielgrößen einzig die Sättigungskonzentration (Sättigungslöslichkeit) des Arzneistoffes sowie die effektiv durch das Lösungsmittel angreifbare Stoffoberfläche. Eine Vergrößerung dieser beiden Parameter sollte auch eine Erhöhung der Lösegeschwindigkeit zur Folge haben.

Klassische Verfahren zur Erhöhung der Sättigungslöslichkeit von Arzneistoffen sind z. B.:

a) die Zugabe von mit Wasser mischbaren organischen Lösungsmitteln, bzw.
b) der Einsatz von hydrotropen Stoffen.

Diese Maßnahmen haben aber den Nachteil, daß sie zum einen den Organismus mit toxikologisch bedenklichen Stoffen belasten, zum anderen kann eine erhöhte Löslichkeit in vivo durch die Rekristallisationsvorgänge zunichte gemacht werden. Oft reicht außerdem die anzuwendende Menge nicht aus, um eine erforderliche Arzneistoffdosis in Lösung zu bringen.

Daher wurde in neuerer Zeit die Solubilisierung von Arzneistoffen durch grenzflächenaktive, mizellbildende Substanzen bzw. die Bildung von Cyclodextrin-Einschlußverbindungen beschrieben. Beide Anwendungen besitzen aber den wesentlichen Nachteil, daß primär gelöster Arzneistoff im Organismus tatsächlich nicht frei vorliegt, sondern aus seinem Komplex mit dem Hilfsstoff freigesetzt werden muß. D. h. insgesamt wird die Arzneistoff-Freigabe so eher verschlechtert als verbessert. Abgesehen davon sind Nebenwirkungen durch grenzflächenaktive Substanzen nicht auszuschließen.

Eine Oberflächenvergrößerung von Arzneistoffen ist durch Mikronisation möglich. Diese Verarbeitung ist jedoch sehr schwierig und aufwendig und hat ihre Grenze bei einer Partikelgröße von ≧ 1 µm. Je kleiner die Pulverpartikel jedoch sind, desto stärker neigen sie zur Aerophilie und der Benetzungsgrad bei Kontakt mit Lösungsmitteln (effektive Oberfläche A) wird gering - die Lösegeschwindigkeit wird eher herabgesetzt. Daher wird fast immer der Zusatz von hydrophilen Trägerstoffen notwendig.

Weitere bekannte Verfahren zur Herstellung kleiner Partikel sind z. B. die folgenden:

Violanto (US-A-4,826,689) beschreibt eine Methode zur Herstellung von kolloid verteilten Partikeln von wasserunlöslichen organischen Verbindungen. Dabei sind aufwendige Testversuche notwendig, um optimale Werte für die Parameter Temperatur, Rührgeschwindigkeit und Zugabegeschwindigkeit der wäßrigen Präzipitationsflüssigkeit zur Lösung

der festen Verbindung im organischen Lösungsmittel zu ermitteln. Denn nur die Einhaltung dieser Vorbedingungen sichert die Bildung der beschriebenen Partikel. Eine nachfolgende Trennoperation soll die Partikel von der organischen Flüssigkeit befreien. Stabilisierungsmaßnahmen erfordern unter Umständen eine recht zeit- und kostenaufwendige Zetapotentialmessung, nach der sich ein Zusatz von viskositätserhöhenden Stoffen bzw. Tensiden zur wäßrigen Präzipitationsflüssigkeit bemißt, der die Partikellaggregation verhindern soll.

Fessi (EP-A-0 275 796) beschreibt die Herstellung eines ebenfalls fein verteilten Systems mit Arzneistoffen, das jedoch ein definiertes Polymer als Trägersubstanz benötigt. Polymer und Arzneistoff werden in einem Lösungsmittel gelöst und mit einem Nichtlösungsmittel wird präzipitiert. Nachträglich müssen Schritte zur Abtrennung der Nanopartikel, z. B. durch Filtration oder Zentrifugation durchgeführt werden. Bei der Herstellung ist außerdem ein Stabilisatorzusatz (Tenside oder ähnliche Träger) notwendig, um die Partikelaggregation zu minimieren.

Bei den obigen Verfahren sind immer komplexe Zusatzstoffe notwendig, deren Einsatz weder gezielt vorherbestimmt werden kann, noch aus den eingangs erwähnten Gründen (toxikologische Risiken) wünschenswert ist. Daher ist eine einfache Herstellung von stabilen Nanosolen mit möglichst wenig Fremdzusätzen entscheidend für pharmazeutisch relevante Anwendungen.

EP-A-349 428 beschreibt die Herstellung von kolloiden Systemen, bei denen eine wäßrige Lösung, die eventuell eine biologische aktive Substanz enthält, unterhalb der Koagulationstemperatur des Proteins hergestellt wird; weiterhin wird eine wäßrige Lösung, eventuell mit einer biologisch aktiven Substanz, oberhalb der Koagulationstemperatur des Proteins hergestellt. Beide Lösungen werden unter Rühren gemischt und man erhält sofort eine kolloidale Suspension aus Protein und aktiver Substanz durch Koagulation (Ausfällung). Das Wasser kann entfernt werden und man erhält ein trockenes Pulver aus Nanopartikeln.

J.J. Marty et al., Pharm. Acta Helv. 53, 1 (1978) S. 17-23 beschreibt die Herstellung von Gelatine-Nanopartikeln, in die auch Wirkstoffe eingeschlossen werden können. Bei der Herstellung dieser Gelatine-Nanopartikel wird zur Desolvatation und Resolvatation eine pH-Justierung vorgesehen. Eine Überführung des Arzneimittels in Nanopartikel wird nicht offenbart.

Der vorliegenden Erfindung liegt daher die Aufgabe zugrunde, die Bioverfügbarkeit von in Wasser schwer löslichen anorganischen und/oder organischen Verbindungen, durch Erhöhung ihrer Lösegeschwindigkeit ohne Zusatz von schädlichen Hilfsstoffen zu verbessern. Diese Aufgabe wird durch das Verfahren gemäß den Ansprüchen 1 sowie durch das Nanosol gemäß dem Anspruch 25 gelöst.

Gegenstand der Erfindung ist weiterhin gemäß einer Ausführungsform des oben genannten Verfahrens eine Modifikation desselben, die dadurch gekennzeichnet ist, daß man als anorganische und/oder organische Verbindung einen in Wasser schwer löslichen Arzneistoff einsetzt. Gemäß einer weiteren Ausführungsform der Erfindung ist das oben genannte Nanosol dadurch gekennzeichnet, daß die anorganische und/oder organische Verbindung ein in Wasser schwer löslicher Arzneistoff ist und das Nanosol pharmazeutisch applizierbar ist.

Gegenstand der Erfindung sind weiterhin pharmazeutische Zubereitungen zur Verwendung bei der Behandlung von Krankheiten z. B. Herz- Kreislauferkrankungen, Rheuma oder Gicht (allgemein rheumatischen oder entzündlichen Erkrankungen), Diabetes, usw. wenn sie einen Arzneistoff, z. B. Nifedipin, Indometacin, Glibenclamid usw. in Form von Nanopartikeln in einem stabilen kolloid-dispersen System mit Gelatine enthalten.

80% aller Diabetiker leiden unter dem Typ II-Diabetes, verursacht durch eine verminderte Produktion von Insulin. Zur Behandlung dieses Typs haben sich Sulfonylharnstoffe, wie z.B. Tolbutamid, als besonders wirksam erwiesen. Weitergehende Forschungstätigkeiten führten zu den sogenannten oralen Antidiabetica der 2. Generation, wie z.B. Glibenclamid, das in seiner blutzuckersenkenden Wirkung beim Menschen das Tolbutamid 300mal übertrifft.

Glibenclamid, 1 - { 4 - [ 2 - ( 5 - Chlor - 2 - methoxybenzamido ) - ethyl ] - phenylsulfonyl } - 3 - cyclohexylharnstoff, $C_{23}H_{28}ClN_3O_5S$, der Formel:

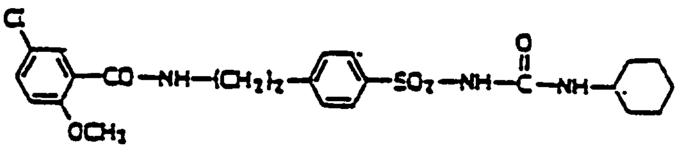

hat sich auf dem Markt weitgehend durchgesetzt. Wie vergleichende in-vivo-Studien belegen, können keine signifikanten Unterschiede hinsichtlich der Bioverfügbarkeitsgröße Serumspiegelfläche (AUC = area under curve) gefunden werden. Gravierende Unterschiede bestehen dagegen jedoch bei den pharmakokinetischen Parametern $c_{max}$ (Maximaler Blutspiegelwert) und $t_{max}$ (der Zeitpunkt, bei dem der maximale Blutspiegelwert erreicht ist). Bei vielen Präparaten kann ein verzögerter Wirkeintritt und eine geringere maximale Blutspiegelkonzentration im Vergleich zu einem Referenzpräparat festgestellt werden. Gerade aber eine schnelle Anflutung des Glibenclamids ist erwünscht. Die akute blutzuckersenkende Wirkung von Glibenclamid ist umso effektiver, je rascher der Wirkstoff im Vergleich zu den Kohlenhydraten systemisch verfügbar ist. Dies hat eine effektive Verminderung und Verkürzung des nahrungsbedingten Anstiegs der Blutglucosewerte zur Folge.

Eine verzögerte Wirkung bei einer Akutformulierung für Glibenclamid kann zwei Ursachen haben:

(1) Die Zubereitung zerfällt nicht schnell genug, so daß der Wirkstoff verzögert freigesetzt wird.

(2) Der Wirkstoff wird im Anschluß an die Freigabe verzögert resorbiert.

Vergleichende in-vitro-Freisetzungsprüfungen zeigen deutliche Unterschiede hinsichtlich der Tablettenzerfallszeiten auf. Einige Tabletten verschiedener Hersteller setzen verzögert frei, beispielsweise werden nach 30 Minuten bei pH 7,4 nur zwischen 50% und 75% des Wirkstoffes freigesetzt. Eine so langsame Freisetzung birgt verschiedene Gefahren in sich, denn dies kann zu Stoffwechselentgleisungen bei Patienten führen, zumal vor allem am frühen Vormittag der Blutglucosewert bekanntermaßen am höchsten ist.

Die zweite Ursache für die mangelnde biopharmazeutische Qualität eines Glibenclamid-Präparats liegt darin begründet, daß der Wirkstoff im Gastrointestinaltrakt, vor allem während der Magenpassage, aufgrund seiner pH-abhängigen Schwerlöslichkeit nicht oder nur vermindert resorbiert wird. Nach der Theorie des passiven Transports können nur Wirkstoffmoleküle resorbiert werden, die gelöst und undissoziiert vorliegen. So beträgt die Löslichkeit von Glibenclamid bei einem pH-Wert von 1,3 1 mg/l, bei pH 6,0 3 mg/l und bei pH 7,8 ca. 30 mg/l (Die Angaben gelten für Raumtemperatur in wäßrigem Milieu). Wie eine Untersuchung an verschiedenen Resorptionsorten im Gastrointestinaltrakt zeigt, erfolgt bei Gabe einer aufgelösten Glibenclamid-Tablette die Resorption am schnellsten im Duodenum.

Nach allgemeiner pharmazeutischer Kenntnis kann die Löslichkeit von Wirkstoffen selbst durch den Einsatz von Tensiden erhöht werden, was aber den entscheidenden Nachteil hat, daß primär gelöster Wirkstoff im Organismus tatsächlich nicht frei vorliegt, sondern aus seinem Komplex (Micelle etc.) freigesetzt werden muß. Dies hat wiederum eine verzögernde Bereitstellung des Wirkstoffs zur Folge. Außerdem steigt das Risiko der Bildung grobkristalliner Anteile durch Rekristallisation. Und darüberhinaus ist der Einsatz von Tensiden aufgrund der bekannten Nebenwirkungen und möglichen Toxizität umstritten.

Wie oben bereits erwähnt, ist die Wasserlöslichkeit von Glibenclamid pH-abhängig. Das bedeutet, daß der Übergang des Wirkstoffs in die resorptionsfähige Form (gelöst und undissoziiert) vom umgebenden pH-Milieu des Gastrointestinaltrakts (GIT) abhängt. Dieser Aspekt ist in zweierlei Hinsicht erwähnenswert. Physiologische pH-Werte, z.B. die der Magenflüssigkeit, können sich einmal von Patient zu Patient unterscheiden. Aber auch individuell kann sich beispielsweise durch Nahrungsaufnahme (leichtes Frühstück, schweres Essen) der pH-Wert der Magenflüssigkeit verändern. Solche inter- und intraindividuellen pH-Wert-Schwankungen führen zu einer unterschiedlichen Bereitstellung von resorptionsfähigen Anteilen und damit zu unterschiedlichen Blutspiegeln. Damit wird der Wirkeintritt, gekennzeichnet durch eine Insulinausschüttung, verbunden mit einem Abbau der Blutglucose, zeitlich (und mengenmäßg) unkalkulierbar, was zu hypo- und hyperglykämischen Stoffwechselzuständen führen kann. Allen handelsüblichen Glibenclamid-Zubereitungen gemeinsam ist, daß sie aufgrund der pH-abhängigen Schwerlöslichkeit des Wirkstoffs von solchen individuellen Unterschieden abhängig sind.

Die Erfindung betrifft weiterhin ein solches Arzneimittel, das 3-Indolylessigsäurederivate, besonders Indometacin oder Acemetacin als Akutform enthält. Schließlich betrifft die Erfindung die Verwendung eines pharmazeutisch applizierbaren Nanosols von 3-Indolylessigsäurederivaten, besonders Indometacin oder Acemetacin, zur Herstellung von Arzneimitteln mit akuter analgetischer und/oder antirheumatischer Wirkung.

Trotz vielfältiger galenischer Entwicklungen peroraler, schnell anflutender Akutzubereitungen ist es bis heute bei handelsüblichen Arzneimitteln, die 3-Indolylessigsäurederivate, besonders Indometacin oder Acemetacin enthalten, noch nicht gelungen, den arzneiformspezifischen Parameter der Wirkstofffreigabe mit anschließender Wirkstoffresorption so optimal an die physiologischen Gegebenheiten (pH-Verhältnisse im Gastrointestinaltrakt, gastrointestinale Verweilzeit von Formlingen, spezifische Resorptionsfenster für bestimmte Wirkstoffe) anzupassen, daß die Hauptanforderung an eine Akutarzneiform erfüllt wird:

Verkürzte Zeit bis zum Auftreten des Plasmaspiegelmaximalwerts ($t_{max}$), z. B. 1 h und weniger

- und als Voraussetzung dafür möglichst schnelle Resorption des Wirkstoffs nach Freisetzung aus der Arzneiform.

Diese Forderung soll eine hohe therapeutische Effizienz bewirken und damit die Patienten-Compliance entscheidend erhöhen.

Das mittlerweile schon fast als klassisches, nichtsteroidales Antiphlogistikum zu bezeichnende Indometacin, (1-(4-Chlorbenzoyl)-5-methoxy-2-methyl-3-indolyl)essigsäure, $C_{19}H_{16}ClNO_4$, stellt eine wirksame entzündungshemmende Substanz dar, die besonders in der Therapie des akuten Rheuma- und Gichtanfalls eine wichtige Rolle spielt. Andere Krankheitsbilder, bei denen die Behandlung mit Indometacin indiziert ist, sind z.B. rheumatoide Arthritis, Spondylitis ankylosans oder Osteoarthritis. Hierfür sind Indometacin-Zubereitungen mit 25 mg und 50 mg Wirkstoffgehalt handelsüblich.

Acemetacin, (1-(4-Chlorbenzoyl)-5-methoxy-2-methyl-3-indolyl)essigsäurecarboxymethylester, $C_{21}H_{18}ClNO_6$, stellt einen Ester des Indometacins dar, der im Stoffwechsel größtenteils zu Indometacin metabolisiert wird. Sein Wirkprofil entspricht weitgehend dem des Indometacins. Ein wesentlicher Unterschied soll jedoch in der besseren Verträglichkeit bestehen, da Acemetacin im Gegensatz zu Indometacin nur schwach ausgeprägte ulcerogene Eigenschaften besitzen soll.

Das Problem, das man bei der Entwicklung schnell anflutender Akutzubereitungen mit den besagten Wirkstoffen zu überwinden hat, stellt sich folgendermaßen dar, wie am Beispiel des Indometacins deutlich gemacht wird:

Indometacin ist eine praktisch wasserunlösliche Wirkstoffsäure mit einem $pK_A$-Wert von 4,5 . Da im allgemeinen nur Stoffe, die im Organismus gelöst und undissoziiert vorliegen, resorbiert werden, ist für Indometacin keine nennenswerte Resorption im sauren Magen-Milieu (pH 1) zu erwarten. Erst im Verlauf der weiteren gastrointestinalen Passage (pH-Erhöhung im Duodenum) löst sich genügend Wirkstoff, so daß die Resorption in Gang kommt. Dementsprechend treten bei handelsüblichen Indometacin-Akutarzneiformen maximale Plasmaspiegel erst nach ca. 1-3 h auf ($t_{max}$). Diese Werte sind mit Vorbehalt zu betrachten, denn aufgrund starker inter- und intraindividueller Schwankungen bei der Magenverweilzeit peroraler, nicht retardierter Zubereitungen (z.B. wegen Art und Menge der aufgenommenen Nahrung) gelangt eine Indometacin-Wirkstoff-Dosis nicht immer zur vorherbestimmbaren Zeit in obigen Resorptionsbereich (sog. Resorptionsfenster). Indometacin stellt also einen solchen Wirkstoff dar, bei dem die Resorptionskinetik stets in Bezug zur gastrointestinalen Verweilzeit der Zubereitung gesetzt werden muß.

Daher sind als Zubereitungen vorwiegend in Hartgelatinekapseln abgefüllte Pellets oder Granulate im Handel, weil diese aufgrund ihres geringen Durchmessers (i.a. 1-1,5 mm) noch relativ schnell den Magen passieren können. In Abhängigkeit von Art und Menge der aufgenommenen Nahrung, Füllungszustand des Magens etc. liegen durchschnittliche Verweilzeiten bei ca. 100 min, können aber auch auf z.B. bis zu 300 min ansteigen.

In Kenntnis dieser Fakten wird es leicht verständlich, daß ein unter Schmerzen leidender Patient noch vor dem Wirkeintritt der ersten Dosis eine zweite oder dritte Dosis einnimmt. Damit steigt natürlich die Gefahr einer Überdosierung an.

Gelatine ist ein aus kollagenhaltigem Material gewonnenes Skleroprotein, das je nach Herstellungsprozeß unterschiedliche Eigenschaften hat. Sie besteht im wesentlichen aus vier Molekulargewichtsfraktionen, die die physikalisch-chemischen Eigenschaften in Abhängigkeit vom Molekulargewicht und prozentualem Gewichtsanteil beeinflussen. Je höher z.B. der Anteil Mikrogel ($10^7$ bis $10^8$ D) liegt, desto höher ist auch die Viskosität der wäßrigen Lösung. Handelsübliche Sorten enthalten bis zu 15 Gewichtsprozent. Die Fraktion der $\alpha$-Gelatine und deren Oligomere (9,5 x $10^4$/ $10^5$ bis $10^6$ D) sind entscheidend für die Gelfestigkeit und liegen üblicherweise zwischen 10 und 40 Gewichtsprozent. Molekulargewichte unterhalb der $\alpha$-Gelatine werden als Peptide bezeichnet und können in herkömmlichen Gelatinequalitäten (niedrigbloomig) bis zu 80 Gewichtsprozent betragen.

Je nach Aufarbeitung des Rohmaterials (saurer oder basischer Aufschluß) erhält man Gelatinen, deren isoelektrische Punkte unterschiedlich sind. Für sauer aufgeschlossene Gelatinen liegt der IEP zwischen 6,3 und 9,5 (Gelatine Typ A), für basisch aufgeschlossene Gelatinen zwischen 3,5 und 6,5 (Gelatine Typ B). Durch die unten angegebenen, speziellen Herstellungsverfahren können auch andere IEP's erzielt werden. Allen Gelatinearten gemeinsam ist jedoch ihr amphoteres Verhalten in wäßrigem Milieu. Bei pH-Werten, die nicht mit dem IEP identisch sind, liegt das Makromolekül immer geladen vor.

In Abhängigkeit von der Herstellungsweise von Gelatine (Ausmaß des Abbaus des nativen Kollagens und saures bzw. alkalisches Aufschlußverfahren) weist Gelatine vom Typ A oder Typ B ein charakteristisches Molekulargewichtsspektrum bzw. Molekulargewichtsverteilung auf. In Tabelle 1 sind die Molekulargewichtsverteilungen von verschiedenen Gelatinetypen bzw. von Kollagenhydrolysaten angegeben, sowie der prozentuale Anteil (Häufigkeit) einzelner Molekulargewichtsbereiche.

Tabelle 1

| Molekulargewichtsverteilung von verschiedenen bekannten Gelatinetypen bzw. von bekannten Kollagenhydrolysaten | | | | | | | |
|---|---|---|---|---|---|---|---|
| Molecular Mass Distribution (kD) | Native Collagen % | Gelatin Type B % | Gelatin Type A % | Collagen hydrolysate Gelita ® Collagel A | Collagen hydrolysate Gelita ® Collagel B | Collagen hydrolysate Gelita ® Sol C | Elastin hydrolysate Gelita ® Gelastin |
| >360 | 100 | 18,0 | 18,0 | 0 | 0 | 0 | 0 |
| 285 | 0 | 7,0 | 9,0 | 0 | 0 | 0 | 0 |
| 145-237 | 0 | 20,0 | 34,0 | 1,0 | 1,5 | 0 | 0 |
| 95 | 0 | 26,0 | 11,0 | 0 | 0 | 0 | 0 |
| 95-50 | 0 | 16,3 | 13,4 | 2,6 | 4,0 | 1,1 | 0 |
| 50-20 | 0 | 7,4 | 9,1 | 18,0 | 14,5 | 0,3 | 0 |
| 20-10 | 0 | 3,9 | 3,8 | 43,0 | 31,5 | 3,7 | 0,2 |
| 10- 5 | 0 | 3,0 | 3,0 | 15,4 | 20,0 | 12,2 | 5,2 |
| 5- 2 | 0 | 0 | 0 | 6,0 | 14,0 | 26,0 | 93,9 |
| 2- 1 | 0 | 0 | 0 | 7,0 | 8,0 | 23,0 | 0 |
| < 1 | 0 | 0 | 0 | 6,5 | 7,0 | 34,0 | 0 |
| MG | 360 | 165 | 185 | 12-18 | 12-18 | 3 | 2-3 |

Man erkennt in den einzelnen Spalten deutlich das Überwiegen eines einzelnen Bereiches im Vergleich zu den übrigen Molekulargewichtsbereichen derselben Gelatine. Dieser Bereich stellt also das Maximum der Molekulargewichtsverteilung dar (es liegt z.B. bei der in der Abbildung aufgeführten Gelatine Typ B bei 95 kD). Der Begriff des "Maximums der Molekulargewichtsverteilung" ist jedoch streng zu trennen von dem Begriff des "durchschnittlichen mittleren Molekulargewichts". Dieser Mittelwert liegt bei der erwähnten Gelatine vom Typ B bei 165 kD.

Kolloidale Dispersionen sind i.a. metastabil und flocken daher aus bzw. sedimentieren. Durch das Überwiegen der destabilisierenden Kräfte, verursacht durch van der Waals-Anziehung, ist die elektrostatische Abstoßung der an der Oberfläche einheitlich geladenen Partikeln zu gering, sodaß größere Partikel auf Kosten der kleineren wachsen, was als Ostwald-Reifung bezeichnet wird.

Überraschenderweise zeigt sich bei der Lösung der oben genannten Aufgabe, daß bei Gelatine die Einstellung ihres Ladungszustandes durch die Protonierung bzw. Deprotonierung relativ zum isoelektrischen Punkt (IEP) völlig ausreichend ist, um erfindungsgemäß eine in Wasser schwer lösliche organische Verbindung, insbesondere einen solchen Arzneistoff in Form eines Nanosols zu stabilisieren.

Im Rahmen der Erfindung hat sich nun gezeigt, daß die geladenen, kolloiden Arzneistoffpartikel dann stabilisiert werden, wenn ein Ladungsausgleich zwischen diesen Partikeln und einer, gegensinnig geladenen Gelatine, einem Kollagenhydrolysat bzw. einem Gelatinederivat erreicht ist. Dieser Zustand ist der isoionische Punkt (IIP). Dabei zeigt sich erstaunlicherweise, daß die Ostwald-Reifung der kolloiden Arzneistoffpartikel gemäß der Erfindung unterbunden wird. Die Partikel liegen nahezu monodispers vor und sind am Wachstum gehindert. Das Gesamtsystem wird dann als erfindungsgemäßes Nanosol bezeichnet.

Fig.1 zeigt eine schematische Darstellung der einstellbaren Ladungszustände von Gelatinen in Abhängigkeit vom pH-Wert und IEP, wobei der IEP je nach Herstellungsart zwischen 3,5 und 9,5 liegen kann. Unterhalb von pH 3,5 sind fast alle Gelatinetypen positiv geladen. Im basischen Bereich oberhalb von pH 9,5 sind alle Gelatinetypen negativ geladen.

Erfindungsgemäß wird daher die Tatsache ausgenutzt, daß Gelatinen, Kollagenhydrolysate oder Gelatinederivate (nahezu unabhängig von der Viskosität) dann zu einem stabilen kolloid-dispersen Systems in Nanosolform führen, wenn der isoionische Ladungszustand zwischen Arzneistoffpartikel und Gelatine, Kollagenhydrolysat oder Gelatinederivat vorliegt.

Dagegen wurde Gelatine nach dem Stand der Technik nur zur Stabilisierung eines anorganischen, kolloid-dispersen Systems eingesetzt. So beschreibt z. B. das DAB 9 eine kolloidale Injektionslösung von radioaktivem Gold, die mit

Gelatine hergestellt ist. Man stellte sich dabei lediglich vor, daß sich das Makromolekül als "Kittsubstanz" zwischen den einzelnen Kolloidpartikeln befinde und so eine Teilchenaggregation verhindert werde. Dagegen war über den Stabilisierungsmechanismus, z.B. für Arzneistoffe, bisher nichts bekannt.

Weitere internationale (PCT)-Patentanmeldungen der ALFATEC-Pharma GmbH, gegebenenfalls auch der PAZ Arzneimittelentwicklungsgesellschaft mbH, von demselben Tage betreffen die Akutform von 2-Arylpropionsäurederivaten (DE-A- 41 40 185), die Retardform von Dihydropyridinderivaten (DE-A-41 40 194), die Akutform von S- und R-Ibuprofen (DE-A-41 40 179), die Retardform von S- und R-Ibuprofen (DE-A-41 40 172), die Akutform von S- und R-Flurbiprofen (DE-A-41 40 184), die Retardform von S- und R-Flurbiprofen (DE-A-41 40 183) und Retardform von Indolylessigsäurederivaten (DE-A-41 40 191).

Der Beladungsgrad der erfindungsgemäßen Nanosole mit Arzneistoff, ausgedrückt in g Arzneistoff pro g Gelatine, Kollagenhydrolysat oder Gelatinederivat, richtet sich im allgemeinen nach der Dosierung für den betreffenden Arzneistoff. Er kann 1 : 200 bis 1 : 0,5 betragen, beträgt üblicherweise 1 : 50 bis 1 : 1, insbesondere 1 : 20 bis 1 : 3. Damit ist das Nanosol erstaunlicherweise geradezu für solche Arzneistoffe sehr gut geeignet, die gewöhnlich hoch dosiert werden müssen, wie z. B. Ibuprofen (Einzeldosis für analgetische Therapie = 200 mg, für Rheumatherapie = 400 mg). Eine erfindungsgemäße Verbesserung der Bioverfügbarkeit bedeutet hier eine Dosisreduktion, deren Tragweite für eine Therapie entscheidend sein kann. Mit weniger Arzneistoff ist somit eine effizientere Therapie mit geringerer toxischer Belastung des Organismus möglich.

Die Arzneistoffpartikel im erfindungsgemäßen Nanosol haben bevorzugt eine durchschnittliche Teilchengröße von 10 bis 800 nm, insbesondere unterhalb von 400 nm.

Der Arzneistoff für die erfindungsgemäßen Nanosole hat bevorzugt eine Löslichkeit in Wasser bei Raumtemperatur von kleiner als 5 g/l, insbesondere kleiner als 1 g/l.

Sofern erforderlich, können übliche pharmazeutische Hilfsstoffe und/oder weitere Makromoleküle unter Beachtung der Stabilität dem erfindungsgemäßen Produkt in flüssigem oder getrocknetem Zustand zugesetzt werden.

Ein Zusatz von z.B. Polyvinylpyrrolidon hat sich technologisch als besonders geeignet erwiesen. Dabei wird insbesondere bei niedrigmolekularem PVP (z.B. PVP K 15) die Stabilität des Nanosols nicht verringert. Das Mengenverhältnis Gelatine zu Polyvinylpyrrolidon kann dabei beispielsweise im Bereich von 5:1 bis 500:1 liegen.

Nanosole können sich für die üblichen Applikationsarten eignen. Z. B. sind die erfindungsgemäßen Nanosole bei Verwendung von kaltwasserlöslicher/modifizierter Gelatine zur Anwendung als Parenteralia geeignet. Modifizierte Gelatine kann z.B. ein handelsüblicher Plasmaexpander sein. Weiterhin können die Nanosole zur pulmonalen Applikation oder zur transdermalen Applikation (z.B. halbfeste Arzneiform) verwendet werden. Insbesondere eignen sie sich jedoch zur sublingualen und zur peroralen Applikation und für Arzneiformen mit bioadhäsiven Eigenschaften.

Ein weiterer Vorteil der vorliegenden Erfindung ergibt sich aus der großen Variationsbreite der verwendbaren Gelatinesorten, die vorteilhafterweise zu vereinfachten technologischen Anwendungen führt. So kann man durch Verwendung von sich schnell auflösenden Gelatinesorten zu Akutformen des Nanosols auf Tablettenbasis gelangen, die fast ausschließlich aus einem Hilfsstoff bestehen (z.B. Direkttablettierung). Darüberhinaus läßt sich ein erfindungsgemäß hergestelltes Nanosol selbst bei Verwendung von hochmolekularen Gelatinequalitäten unproblematisch sprüh- oder gefriertrocknen.

Sprühgetrocknete Nanosole ergeben ein leicht dosierbares bzw. granulierbares Pulver, das zu peroralen Arzneiformen wie z.B. Hartgelatinekapseln, Granulaten/Pellets oder Tabletten weiterverarbeitet werden kann.

Werden die erfindungsgemäßen Nanosole (mit oder ohne übliche Gerüstbildner) lyophilisiert, lassen sich besonders schnell freisetzende Arzneiformen entwickeln, wobei Gelatinen mit hohem Peptidanteil bevorzugt sind.

Für die Entwicklung von peroralen Retardarzneiformen lassen sich vorteilhaft Retard-Nanosole darstellen, wie sie beispielsweise in der internationalen (PCT)-Patentanmeldung PCT/DE92/01011 mit dem Titel "Sol-gesteuerte Thermokolloidmatrix auf Gelatinebasis für perorale Retardarzneiformen" desselben Anmelders vom selben Tag beschrieben werden.

Einzelheiten über spezielle Arzneiformen, die schwer lösliche Wirkstoffe in Akut- und/oder Retard-Nanosolform enthalten, können aus den bereits erwähnten Anmeldungen entnommen werden.

Bei der Formulierung von Akut- bzw. Retardpräparaten macht der Pharmazeut einen grundsätzlichen Unterschied zwischen:

1. galenischer Zubereitung, d.h. einer Freisetzung des Arzneistoffes, z.B. aus einer Tablette in zeitlich schneller (Akutform) oder verlangsamter (Retardform) Weise;
und

2. dem arzneistoffspezifischen Resorptionsort, wie z.B. Magen oder bestimmte Darmabschnitte.

Die erfindungsgemäßen Nanosole sind in der Lage, unabhängig von der galenischen Zubereitung, aufgrund ihrer speziellen Zusammensetzung, im gesamten gastrointestinalen Bereich resorbiert zu werden. Sie können daher vorteilhaft zu Akut-bzw. Retardarzneiformen weiterverarbeitet werden.

Fig. 2 zeigt den Mechanismus der passiven Arzneistoffresorption im Gastrointestinal-Trakt.

Weiterhin eignen sich für die erfindungsgemäßen Nanosole Arzneistoffe mit problematischer Bioverfügbarkeit, insbesondere,

1. aus der Gruppe der starken Analgetika, z. B. Morphin, Dextropropoxyphen, Pentazocin, Pethidin, Buprenorphin;

2. aus der Gruppe der Antirheumatika/Antiphlogistika (NSAR), z. B. Indometacin, Diclofenac, Naproxen, Ketoprofen;

3. aus der Gruppe der β-Sympathicolytica, z. B. Propranolol, Alprenolol, Atenolol, Bupranolol;

4. aus der Gruppe der Steroidhormone, z. B. Betamethason, Dexamethason, Methylprednisolon, Fludrocortison und Ester, Ethinylestradiol, Medroxyprogesteronacetat;

5. aus der Gruppe der Tranquillizer, z. B. Oxazepam, Diazepam;

6. aus der Gruppe der α-Sympatholytica, z. B. Dihydroergotamin;

7. aus der Gruppe der Hypnotika, z. B. Secbutabarbital, Secobarbital, Pentobarbital;

8. aus der Gruppe der tricyclischen Antidepressiva, z. B. Nortriptylin, Clomipramin, Amitryptilin;

9. aus der Gruppe der Neuroleptika, z. B. Chlorprothixen, Chlorpromazin, Haloperidol, Trifluopromazin;

10. aus der Gruppe der Gichtmittel, z. B. Benzbromaron, Allopurinol;

11. aus der Gruppe der Antiparkinsonmittel, z. B. Levodopa;

12. aus der Gruppe der Koronartherapeutika oder Calciumantagonisten z. B. Nifedipin u. a. Dihydropyridinderivate, Gallopamil;

13. aus der Gruppe der Antihypertensiva, z. B. Clonidin, Methyldopa, Dihydralazin, Diazoxid, Renin-Antagonisten;

14. aus der Gruppe der Diuretika, z. B. Mefrusid, Hydrochlorothiazid, Furosemid, Triamteren, Spironolacton;

15. aus der Gruppe der oralen Antidiabetika, z. B. Tolbutamid, Glibenclamid;

16. Peptidarzneistoffe, z.B. Insulin, Renin-Antagonisten.

17. Digitalisglykoside;

18. Antiarrhytmika;

19. Antibiotika/Chemotherapeutika, z.B. Nitrofurantoin;

20. Antiepileptika;

21. Antikoagulantia;

22. Spasmolytika;

23. Antimykotika;

24. Hormone;

25. Venentherapeutika;

26. Immunsuppressiva, z.B. Ciclosporin;

27. Tuberkulostatika;

28. Virustatika;

29. Zytostatika;

30. Provitamine und Vitamine;

31. Phytopharmaka

32. aus der Gruppe der Arzneistoffe zur Behandlung von erworbener Immunschwäche (AIDS).

Im Sinne der oben genannten Arzneistoffliste sind auch enantiomerreine Wirkstoffe oder Pseudoracemate erfindungsgemäß geeignet.

Weiterhin können die erfindungsgemäßen Nanosole für Wirkstoffe aus dem Bereich der diätetischen Lebensmittel verwendet werden.

Es hat sich erstaunlicherweise im Rahmen der vorliegenden Erfindung gezeigt, daß sich eine Vielzahl von Arzneistoffen in Nanosolform überführen lassen, wenn nach Auswahl einer geeigneten Gelatine (Typ A oder B mit charakteristischem isolelektrischem Punkt, Molekulargewicht, usw.) eine solche Nettoladung des Moleküls eingestellt wird, die zur Ladungsneutralität (isoionischer Punkt = IIP) mit den geladenen Arzneistoffpartikeln führt und eine für den Arzneistoff (Säure, Base oder Neutralstoff bzw. amphoterer Stoff) geeignete erfindungsgemäße Herstellung wählt.

Das erfindungsgemäß erhaltene Produkt verhält sich pharmazeutisch gesehen wie eine echte Lösung, ohne jedoch die Probleme des Standes der Technik aufzuweisen; d. h. auf pharmakologisch bedenkliche Hilfsstoffe kann verzichtet werden.

Überraschenderweise zeigt sich, daß das Vorliegen stabiler Nanopartikel z.B. im Falle des Glibenclamids oder der schwer wasserlöslichen 3-Indolylessigsäurederivate, besonders Indometacin oder Acemetacin, völlig ausreichend ist, eine Arzneistoffresorption zu erreichen, die

a) unmittelbar auf die Wirkstofffreisetzung aus seiner Zubereitung im Magen erfolgt;
b) unabhängig von den oben geschilderten physiologischen Bedingungen ist;
c) unabhängig von den physikalisch-chemischen Eigenschaften der Wirkstoffsäure ist;
d) nahezu vollständig ist und
e) ohne vorgelagertes Gleichgewicht der Wirkstoffauflösung erfolgt wie bei herkömmlichen Präparaten (der Wirkstoff steht in resorptionsfähiger Form unmittelbar an jedem beliebigen Resorptionsort zur Verfügung).

Damit läßt sich bei unterschiedlichstem Wirkstoff eine Bioverfügbarkeit und Anflutung erreichen, wie sie bisher nicht bekannt ist. Damit verbunden ist ebenso eine Verkürzung der Zeit von der Applikation bis zum Erreichen der Plasmawirkstoffkonzentration im therapeutischen Niveau. Außerdem wird die in der erfindungsgemäßen Arzneiform enthaltene Wirkstoffdosis vollständig ausgenutzt, sodaß damit insgesamt gesehen eine Dosisverminderung gegenüber konventionellen Präparaten bei vergleichbarer Wirkung zustande kommt. Erstaunlicherweise hat sich nämlich gezeigt, daß diese Nanopartikel in dem erfindungsgemäßen Nanosol an jedem gewünschten Resorptionsort ungehindert die Gastrointestinalmembran passieren können (resorbiert werden). Sie verhalten sich also, biopharmazeutisch gesehen, wie eine echte Lösung, ohne aber eine solche zu sein.

Es hat sich erstaunlicherweise gezeigt, daß nur Nanopartikel, deren Größe im Bereich von 10 - 800 nm liegt, bevozugt unterhalb 400 nm direkt resorbiert werden können. Diese Bedingungen werden durch die erfindungsgemäßen Nanosole Glibenclamid und mit 3-Indolylessigsäurederivaten, besonders Indometacin oder Acemetacin als Wirkstoff, erfüllt.

Die Vorteile dieses neuartigen Produktes liegen damit auf der Hand. Durch eine kontrollierte Resorption der Wirkstoffe bereits im Magen kann die aufgrund ihrer Schwerlöslichkeit bisher als problematisch eingestufte Anflutungsgeschwindigkeit und Bioverfügbarkeit von Glibenclamid und 3-Indolylessigsäurederivaten, besonders Indometacin oder Acemetacin überraschenderweise erheblich verbessert werden unter gleichzeitiger Erhöhung der Verträglichkeit.

Die erfindungsgemäß eingesetzten Nanosole zeichnen sich durch hohe Stabilitäten, insbesondere im sauren Bereich aus, ohne zu flocken oder auszukristallisieren. Das bedeutet, daß das Nanosol ausreichend lang während der Magenverweilzeit und unabhängig von auftretenden pH-Schwankungen, z. B. durch Nahrungseinfluß, der Magenmucosa für die Resorption zur Verfügung steht.

Bei pH-Werten unterhalb von 2 kann die Stabilität des Nanosols durch Auswahl einer auf diesen pH-Bereich abgestimmten Gelatinesorte noch verbessert werden.

Die Teilchen der Nanosole liegen nach ihrer Herstellung, nach Resuspendierung des getrockneten Pulvers und nach Resuspendierung aus einer Arzneiform in Teilchengrößen von 10 bis 800 nm, bevorzugt unterhalb 400 nm und darüberhinaus nahezu monodispers vor. Weiterhin ist das Nanosol im resuspendierten Zustand als Nanodispersion im Magen gut verteilt, was optimale Voraussetzungen für die Resorption schafft. Da die Nanopartikel stabilisiert vorliegen,

können sie als solche resorbiert werden, ohne daß sie vorher aufgelöst werden müssen. Damit entfällt ein zeitlich vorgelagertes Lösungsgleichgewicht wie bei mikronisierten Pulvern oder wasserlöslichen Salzen in jedem Falle. Sie verhalten sich demnach, biopharmazeutisch gesehen wie eine echte Lösung, ohne aber eine solche zu sein.

Somit wird erstmals durch die vorliegende Erfindung eine kontrollierte Resorption im Gastrointestinaltrakt bereits während der Magenverweilzeit möglich. Die Resorption ist nicht mehr auf den Dünndarmbereich beschränkt; es wird eine schnelle Anflutung für Glibenclamid und für 3-Indolylessigsäurederivate, besonders Indometacin oder Acemetacin ermöglicht.

Damit ist es überraschend möglich, bei diesen Arzneistoffen erstmals einen $t_{max}$-Wert unterhalb von 1 h, insbesondere unterhalb von 30 min, zu erreichen.

Zusätzlich läßt sich auch eine Erhöhung des Blutspiegelmaximalwertes $c_{max}$ feststellen. Die Erhöhung von $c_{max}$ kann daher u.U. eine Dosisreduktion bei gleicher Wirksamkeit zur Folge haben.

Wie in-vitro Versuche gezeigt haben, ist aufgrund der aufgeführten langen Stabilitäten der erfindungsgemäßen Nanosole die Gefahr der Rekristallisation im Magen auszuschließen.

Weiterhin kann die Akutform von Glibenclamid bzw. von 3-Indolylessigsäurederivaten auch mit einer Retardformulierung für Glibenclamid bzw. 3-Indolylessigsäurederivate kombiniert werden.

Als besondere Ausführungsform kann ein pulverförmiges oder granuliertes Akut-Nanosol mit einer Matrix-Tablette, wie sie in der oben genannten internationalen (PCT)-Patentanmeldung mit dem Titel "Sol-gesteuerte Thermokolloidmatrix auf Gelatinebasis für perorale Retardformen" der ALFATEC-Pharma GmbH vom selben Tag beschrieben wird, z.B. in einer Hartgelatinekapsel kombiniert werden.

Eine solche Arzneiform setzt den Wirkstoff zunächst schnell frei und die Erhaltungsdosis (Matrix-Tablette) mit hoher Reproduzierbarkeit konstant nach einem Geschwindigkeitsgesetz nullter Ordnung.

Das getrocknete Nanosol kann zu Arzneiformen, beispielsweise zu einer Tablette, weiterverarbeitet und daraus resuspendiert werden, Somit wird ein magensaftresistenter Überzug zum Schutz vor "Inaktivierung" der Wirkstoffe durch den sauren Magen-pH überflüssig.

Die Gefahr einer Überdosierung durch Mehrfacheinnahme wird durch den schnellen Eintritt des therapeutischen Effektes als Folge der Resorption im Magen ausgeschlossen. Alle Nachteile und Gefahren eines magensaftresistenten Überzugs entfallen. Somit dient die vorliegende Erfindung auch der Erhöhung der Patienten-Compliance. Dies alles stellt einen entscheidenden Beitrag zur geforderten Arzneimittelsicherheit dar.

Grundsätzlich läßt sich das erfindungsgemäße Produkt zu allen peroral zu applizierenden Arzneiformen verarbeiten, insbesondere kann es direkt als Pulver in Hartgelatinekapseln abgefüllt werden. Es eignet sich auch hervorragend zur Direkttablettierung. Eine Verarbeitung zu einem Trinkgranulat, schnellauflösenden Pellets oder Trinktabletten ist für die Applikation als schnellanflutende Akutform von besonderem Interesse.

Um die physiologischen Hintergründe der Resorption von Arzneistoffen im allgemeinen und die verbesserte Resorptionsquote der erfindungsgemäßen Nanosole ausreichend zu erläutern, ist zunächst eine Betrachtung zum Mechanismus der physiologischen Resorption von Arzneistoffen erforderlich, wie er auch in einschlägigen Publikationen dargestellt wird. Allerdings ist die vorliegende Erfindung weder an den folgenden Versuch einer wissenschaftlichen Erklärung der erfindungsgemäß auftretenden Phenomene gebunden noch kann sie hierdurch eingeschränkt werden.

Die passive Arzneistoffresorption erfolgt nach heutigem Erkenntnisstand (Theorie nach Brodie et al.), wenn folgende Bedingungen vorliegen:

a) die Gastrointestinalmembran wirkt als Lipidbarriere,
b) der Arzneistoff wird nur in gelöster und ungeladener, d.h. nichtionisierter Form aufgenommen,
c) saure Arzneistoffe werden bevorzugt im Magen, basische Arzneistoffe bevorzugt im Darm resorbiert.

Nach der peroralen Aufnahme eines Arzneistoffs in den Organismus wird seine Resorption, d.h. der Übertritt in den allgemeinen Kreislauf (Biophase) in starkem Maße durch physikalische Barrieren behindert (siehe Fig. 2), nämlich

- durch die Mucus-Schicht und eine wässerige, daran adhärierende Schicht
- die Zellmembranen der intestinalen Epithelzellen mit der daran kovalent gebundenen Glykocalix sowie
- die sogenannten "Tight Junctions", die die Epithelzellen an ihrer apikalen Seite miteinander verbinden.

Diese Barrieren bedingen, daß die Resorption von Arzneistoffen hauptsächlich abhängig von ihrem Verteilungsmechanismus und Ladungszustand- durch die Lipid-Doppelschichten erfolgt (sogenannte passive Diffusion).

Die Epithelzellen des gesamten Magen-Darm-Traktes sind mit einer Mucus-Schicht bedeckt, die aus Mucinen (Glykoproteinen), Elektrolyten, Proteinen und Nucleinsäuren besteht. Vor allem die Glykoproteine bilden mit dem Hauptanteil des Mucus, nämlich Wasser, eine viskose Gelstruktur, die in erster Linie Schutzfunktionen für die darunter liegende Epithelschicht ausübt. Die Mucusschicht ist an die apikale Oberfläche der Epithelzellen über die Glykocalix gebunden.

Die Glykocalix hat ebenfalls eine Glykoproteinstruktur, die kovalent an Bausteine der Membran-Doppelschicht der Epithelzellen gebunden ist. Die verzweigten Polysaccharide der Glykocalix, die entweder direkt an amphiphile Moleküle der Doppelmembran oder an die Doppelmembran inkorporierte Proteine kovalent gebunden sind, besitzen geladene N-Acetyl-Neuraminsäure- und Sulfat-Reste und sind daher negativ geladen, was zu einer elektrostatischen Bindung oder Abstoßung von geladenen Arzneistoffmolekülen bzw. von elektrostatisch geladenen Partikeln führen kann. Die Epithelzellmembranen bestehen aus Phospholipid-Doppelschichten, in die Proteine über ihre hydrophoben Bereiche verankert sind. Die Phospholipid-Doppelschichten mit ihrem lipophilen Anteil stellen eine weitere Barriere für den Transport der zu resorbierenden Arzneistoffe dar.

Aus dieser Darstellung geht deutlich hervor, daß geladene Arzneistoffmoleküle bzw. elektrostatisch geladene Partikel daher nur eine sehr geringe Chance haben, über den peroralen Applikationsweg resorbiert zu werden,

Die erfindungsgemäßen Nanosole geben erstmalig die technische Lehre, ein System zu bilden, mit dem diese vorgenannten Resorptionshindernisse zu überwinden sind. Da die Wirkstoff-Nanopartikel durch die Gelatine erfindungsgemäß ladungsneutral stabilisiert werden, kann ihr Transport durch die negativ geladene Glykocalix ohne größere Hindernisse erfolgen, im Gegensatz zu sonstig beschriebenen Nanopartikeln des Standes der Technik, die nicht ladungsneutral stabilisiert werden bzw. stabilisiert werden können. Erfindungsgemäß kann die Einstellung des isoionischen Ladungszustandes zusätzlich noch in Abstimmung auf die physiologischen Verhältnisse erfolgen.

Da die erfindungsgemäßen Wirkstoff-Nanosole die Glykocalix ungehindert passieren können, ohne durch elektrostatische Effekte gebunden bzw. abgestoßen zu werden, erreichen sie damit auch die Oberfläche der Epithelzellen und stehen dort in hoher Konzentration zur Verfügung.

Nun können auch aktive, carriervermittelte Transportmechanismen bzw. Phagozytose einen wesentlichen Beitrag zur Resorption der Wirkstoff-Nanosole liefern.

Erfindungsgemäß werden also vor allem folgende Vorteile gegenüber dem Stand der Technik erzielt:

- in Wasser schwer lösbare anorganische und organische Verbindungen werden in eine Form mit neuen Eigenschaften gebracht;
- das Verfahren ist auf nahezu alle schwerlöslichen anorganischen und organischen Verbindungen anwendbar;
- es ist einfach und ohne aufwendige Geräte und Apparaturen durchzuführen;
- im Körper schlecht lösliche bzw. resorbierbare Arzneistoffe können so in eine Form überführt werden, die sich wie eine echte Lösung verhält;
- dies gelingt ohne chemische Veränderung, z. B. ohne die Bildung eines Derivates, oder Bildung eines chemischen Komplexes;
- dies gelingt ohne Zusatz von grenzflächenaktiven oder hydrotropen Substanzen;
- in dieser Form sind schwer lösbare Verbindungen in vivo schneller und vollständiger resorbierbar;
- die Dosierung kann reduziert werden;
- die erhaltene Form ist lagerstabil;
- das Biopolymer Gelatine oder ihr Derivat ist ein toxikologisch unbedenklicher Hilfsstoff;
- Gelatine in Akut- bzw. Retardformen trägt zu einer guten Verträglichkeit des erfindungsgemäß formulierten Arzneistoffs bei;
- die angegebenen Herstellungsverfahren sind wirtschaftlich.

Das erfindungsgemäße System ist von individuellen Unterschieden, was pH-Wert-Schwankungen oder pH-Einflüsse z.B. durch Nahrungsmittel betrifft, vollkommen unabhängig. Ein zeitlich (und mengenmäßig) unkalkulierbarer Wirkeintritt, wie es bei Produkten des Standes der Technik der Fall sein kann, ist damit ausgeschlossen, das Risiko von Nebenwirkungen ist reduziert. Somit stellt die vorliegende Erfindung einen entscheidenden Beitrag zur geforderten Arzneimittelsicherheit dar.

Grundsätzlich läßt sich das erfindungsgemäße Produkt zu allen peroral zu applizierenden Arzneiformen verarbeiten, insbesondere kann es direkt als Pulver in Hartgelatinekapseln abgefüllt werden. Es eignet sich auch hervorragend zur

Direkttablettierung. Eine Verarbeitung zu einem Trinkgranulat, schnellauflösenden Pellets oder Trinktabletten ist für die Applikation als schnellanflutende Akutform von besonderem Interesse.

Prinzipiell eignen sich zur Herstellung der erfindungsgemäß verwendeten Nanosole alle in der vorliegenden Anmeldung der ALFATEC-Pharma GmbH genannten Vorgehensweisen und Verfahrensvarianten und die Herstellung von Gelatine (Beispiel I bis III). Im Falle der Akutform für Glibenclamid sei als bevorzugt geeignetes Verfahren für die Nanosol-Herstellung die Variante Nr. II und III genannt (siehe unten).

Gelatine ist ein aus kollagenhaltigem Material gewonnenes Skleroprotein, das je nach Herstellungsprozeß unterschiedliche Eigenschaften hat. Es existieren Molekulargewichtsbereiche von einigen Tausend D bis hin zu einigen Millionen D, die in ihrer Molekulargewichtszusammensetzung und in ihrem physikalisch-chemischen Verhalten höchst unterschiedlich sein können. Bei genauer Kenntnis dieser Zusammenhänge lassen sich neue pharmazeutische Anwendungen finden, die sich durch hohe Reproduzierbarkeit und einfache technologische Verarbeitung auszeichnen. Einzelheiten können aus den o.g. Anmeldungen entnommen werden. Bei besonders schonender Herstellungsweise kann man Gelatinesorten erhalten, die nur einen geringen Anteil an rechtsdrehenden Aminosäuren aufweisen und somit ähnlich aufgebaut sind wie das native Kollagenmolekül. Diese Gelatinen zeichnen sich zum Beispiel durch besonders gute Stabilisierungseigenschaften für Nanosole aus. Eine solche Gelatine ist erfindungsgemäß vorteilhaft geeignet. Je nach Aufarbeitung des Rohmaterials (saurer oder basischer Aufschluß) erhält man Gelatinen, deren isoelektrische Punkte ganz unterschiedlich sind. Durch spezielle Herstellungstechniken können isoelektrische Punkte gezielt hergestellt werden, wobei die Molekulargewichtsverteilung auf den Anwendungsfall abgestimmt sein kann.

Im Falle von Glibenclamid sind bevorzugt Gelatinesorten geeignet, deren Maximum der Molekulargewichtsverteilung unterhalb $10^5$ D liegt. Zur Tablettenherstellung, wie sie üblicherweise bei oralen Antidiabetica im Vordergrund steht, eignen sich bevorzugt Gelatinesorten mit Bloomwerten von 0 - 50 und einem Maximum der Molekulargewichtsverteilung im Bereich von $10^4$ - 9,5 x $10^4$ D.

Bei den genannten Gelatinen kann ein Gewichtsverhältnis Gelatine zu Wirkstoff von 1:1 bis 200:1 eingestellt werden, wobei ein höheres Gewichtsverhältnis vorteilhafterweise bei der Verarbeitung zu Tabletten etc. zur Vermeidung weiterer Hilfsstoffe gewählt werden kann (z.B. Direkttablettierung).

Für die erfindungsgemäß verwendeten Nanosole mit Glibenclamid oder Indometacin eignen sich auch handelsübliche Gelatinen, fraktionierte Gelatine, Kollagenhydrolysate und Gelatinederivate, insbesondere solche Sorten, die durch eine niedrige Bloomzahl von 0 (kaltwasserlösliche Gelatine oder Kollagenhydrolysate) bis 240 Bloom, vorzugsweise 0 bis 170 Bloom charakterisiert sind.

Im Falle des Glibenclamids werden bevorzugt Gelatinesorten mit IEP's von 3,5 bis 7,5 eingesetzt.

Für die Sprüh- oder Gefriertrocknung von Glibenclamid-Nanosolen hat sich ein Zusatz von Polvinylpyrrolidon (PVP) zur wäßrigen Gelatinelösung, insbesondere PVP K 15 oder PVP K 25 im Gewichtsverhältnis von 1:5 bis 1:30 als vorteilhaft gezeigt, wobei ohne negative Beeinflussung der Stabilität des Nanosols ein gut rieselfähiges Pulver erhalten wird.

Prinzipiell eignen sich für die Herstellung der erfindungsgemäßen Nanosole auch handelsübliche Gelatinen, stark abgebaute Gelatinen (Kollagenhydrolysate bzw. kaltwasserlösliche Gelatine), modifizierte Gelatinen und fraktionierte Gelatine (Einzelfraktionen bzw. deren Mischung).

Ein zu hoher Anteil an Fremdionen (Aschegehalt > 2%) kann sich jedoch störend auswirken und sollte durch Entsalzung mit Ionenaustauschharzen entfernt werden (s. allgemein zur Gelatine: Ullmann, Encyclopädie der technischen Chemie, 3. Aufl. 1954 Bd. 10 und 4. Aufl. 1976 Bd. 12, S. 211; H.E. Wunderlich: Wenn es um Gelatine geht - Herausgeber: Deutscher Gelatine-Verbraucherdienst, Darmstadt (1972); I. Tomka, Gelatine, in: W. Fahrig, U. Hofer, Die Kapsel, Wissenschaftliche Verlagsgesellschaft mbH Stuttgart, 1983, S. 33-57.

Gegenüber handelsüblichen Produkten führt die Verwendung von Gelatine, die auf spezielle Weise hergestellt wurde, zu erfindungsgemäß beschriebenen Nanosolen mit erhöhter Stabilität.

Beispiele für die Herstellung erfindungsgemäß besonders geeigneter Gelatinequalitäten werden unten gegeben.

**Beispiele für die Herstellung von erfindungsgemäß besonders geeigneten Gelatinesorten mit isoelektrischen Punkten von 3,5 bis 9,5**

Beispiel I:

**Verfahren zur Erzielung eines IEP's von 7,5 bis 9,5**

Kollagenhaltiges Ausgangsmaterial wie z.B. Schweineschwarten werden mit einer wäßrigen Lösung einer 0,45 N Mineralsäure, vorzugsweise Schwefelsäure, im Flottenverhältnis 1:1 12 bis 20 Stunden behandelt. Anschließend wird der Säureüberschuß durch mehrmaliges Waschen entfernt, wobei zur Abkürzung des Verfahrens Natriumhydrogencarbonat verwendet werden kann. Die Extraktion des sudreifen Materials erfolgt mit heißem Wasser bei 55 - 80° C bei einem pH von 2,5 bis 4,5. Bei pH-Werten unterhalb von 3,5 kann ein IEP von 8,5 bis 9,5 erreicht werden, bei pH-Werten oberhalb 3,5 liegt der IEP bei 7 bis 8,5. Auf diese Weise lassen sich verschiedene IEP's von 7 bis 9,5 in direkter Abhängigkeit vom pH-Wert während der Extraktion erzielen.

Nach der Verfahrensstufe der Extraktion wird die wäßrige Lösung neutralisiert und wie üblich aufgearbeitet.

Durch dieses Verfahren kann man weiterhin in Abhängigkeit von der gewählten Temperatur während der Extraktion Gelatinesorten mit hohen bis mittleren Molekulargewichtsverteilungen erhalten.

Bei Temperaturen von 50-55° C erhält man besonders hochviskose und hochbloomige Qualitäten. Gelatinesorten mit niedrigem Molekulargewicht bzw. kaltwasserlösliche Gelatinen können durch gezielten Abbau mit Kollagenasen erhalten werden.

Beispiel II:

**Verfahren zur Erzielung eines IEP's von 4 bis 7,5**

Das kollagenhaltige Ausgangsmaterial wird zur Entfernung von Fremdstoffen zunächst gewaschen, zerkleinert und anschließend durch Zusatz von Magnesit, Natronlauge oder Calciumhydroxid durch gründliches Vermischen im Flottenverhältnis 1:1,2 homogen alkalisch gemacht. Das so vorbehandelte Material wird kurzzeitig druckhydrolytisch bei 1,01 x $10^5$ bis 2,02 x $10^5$ Pa und einem pH-Wert der wäßrigen Lösung von 8-14 aufgeschlossen. Nach dem Aufschluß wird sofort neutralisiert und die noch heiße wäßrige Gelatinelösung wie üblich filtriert, entsalzt, aufkonzentriert und getrocknet.

Nimmt man ein schwach basisches Aufschlußmittel wie Magnesit, erhält man einen IEP von 6 bis 7,5, sofern man bei 1,01 x $10^5$ Pa arbeitet. IEP's von 5 bis 6 erhält man bei Einsatz einer verdünnten Kalkmilchsuspension und bei Verwendung von 0,005 bis 0,1 N Natronlauge können IEP's von 4 bis 5 erzielt werden.

Gelatinesorten mit geringem Racemisierungsgrad und niedrigem Peptidanteil lassen sich bei Druckverhältnissen von 1,01 x $10^5$ Pa und Verweilzeiten von maximal 10 Min. erreichen.

Mittel- bis niedrigmolekulare bis hin zu kaltwasserlöslichen Sorten ergeben sich durch entsprechend längere Verweilzeiten.

Beispiel III:

**Verfahren zur Erzielung eines IEP's von 3,5 bis 6**

Kollagenhaltiges Ausgangsmaterial, vorzugsweise Spalt bzw. Ossein, wird nach der Eingangswäsche einem Kurzzeitäscher unterworfen. Hierbei bieten sich zwei Verfahrensvarianten im Flottenverhältnis 1:1,3 an, die entweder eine gesättigte Kalkmilchsuspension oder eine 0,1 bis 1 N Natronlauge zum Einsatz bringen.

Bei Verwendung einer Kalkmilchsuspension wird das Rohmaterial unter ständiger Bewegung maximal 3 bis 4 Wochen aufgeschlossen. Anschließend wird das Material durch Säurezugabe neutralisiert und mehrmals gewaschen. Die weitere Aufarbeitung folgt wie üblich. Auf diese Weise lassen sich IEP's von 4 bis 6 einstellen.

Bei Einsatz von Natronlauge läßt sich der Äscherprozeß nochmals verkürzen, wobei bei Konzentrationen von 1 N Natronlauge das Material je nach Zerkleinerungsgrad bereits nach 6 - 12 Stunden aufgeschlossen ist. Die Neutralisation erfolgt mit äquimolaren Mengen Mineralsäure und die Neutralsalze werden durch mehrmaliges Waschen oder durch Entsalzen der in der Extraktion gewonnenen wäßrigen Gelatinelösung entfernt. Bei dieser Verfahrensvariante lassen sich IEP's von 3,5 bis 5 erhalten.

Besonders peptidarme Gelatinesorten werden bei kurzer Verweilzeit im Äscher erhalten. Man kann so Gelatinesorten mit hoher bis mittlerer Molekulargewichtsverteilung (M = $10^4$ - $10^7$ D) erhalten.

Niedrigmolekulare bis kaltwasserlösliche Gelatinesorten kann man durch thermischen Abbau bzw. enzymatisch erhalten.

Wie eingangs schon erwähnt und wie aus Fig.1 ersichtlich ist, hängt die absolute, maximal mögliche Nettoladung eines einzelnen Gelatinemoleküls hauptsächlich von der Anzahl der freien COOH- und $NH_2$-Gruppen und dem pH-Wert der Lösung ab. Da sich Typ A, B, Kollagenhydrolysate oder Gelatinederivate in der Anzahl freier COOH-Gruppen unterscheiden, ist damit auch ihre maximal mögliche Nettoladung unterschiedlich. Bei Gelatinederivaten kann der Ladungszustand zusätzlich von der Art der Modifizierung abhängen.

Bei der Durchführung des erfindungsgemäßen Verfahrens wählt man in einem Vortest die geeignete Gelatine und den geeigneten pH-Wert aus.

Zunächst wird ein den physikalisch-chemischen Eigenschaften des Arzneistoffs angepaßter Arbeits-pH-Bereich gewählt. Als physikalisch-chemische Eigenschaft des Arzneistoffs sind vor allem zu berücksichtigen Die Löslichkeit (in organischen Lösungsmitteln bzw. Wasser), seine Eigenschaft als Säure, Base oder Neutralstoff sowie seine Stabilität gegenüber Säuren und Laugen.

In einem ersten Schnelltest wird festgestellt, weiche Ladung die ausgefällten Partikel besitzen. Daraus ergibt sich, unter Berücksichtigung des Arbeits-pH-Bereichs, die Wahl eines geeigneten Gelatinetyps. Sind die Teilchen beispielsweise negativ geladen, sucht man eine Gelatine aus, die unter den gegebenen pH-Bedingungen positiv geladen ist. Dieser Schnelltest zur Feststellung der Partikelladung hat die Vorteile, daß er ohne großen apparativen und zeitlichen

Aufwand durchgeführt werden kann. So kann auf eine zeitaufwendige und ungenaue Zeta-Potential-Messung gänzlich verzichtet werden.

In vielen Fällen wird es ausreichend sein, für diesen Schnelltest zwei handelsübliche Gelatinen Typ A und B mit einem IEP von 9,5 bzw. 3,5 mit Peptidanteilen <30 % und einer Bloomzahl von 200, die weiterhin als Standardgelatinen bezeichnet werden, bei einem pH-Wert von 6 in die Solform zu überführen (5%ige wäßrige Lösung) und den Arzneistoff in einem mit Wasser mischbaren Lösungsmittel, wie z. B. Ethanol, Isopropanol oder Aceton, zu lösen und jeweils mit den Gelatinelösungen homogen zu mischen. Bei gleicher Dosierung des Arzneistoffs wird sich bei der in ihrem Ladungszustand nicht geeigneten Gelatine ein kolloidales System entweder nicht ausbilden oder sofort instabil werden bzw. der Arzneistoff ausflocken. Sind die entstehenden Partikel negativ geladen, werden sie eher von Gelatinelösung mit Typ A, der bei einem pH-Wert von 6 positiv geladen ist, stabilisiert als von der Lösung mit Gelatine Typ B; im Gegenteil wird in diesem Fall Typ B entweder kein kolloidales System ausbilden oder das System sofort destabilisieren. Das Ausflocken der Teilchen läßt sich z. B. über eine einfache Trübungs-Messung verfolgen.

Bei diesem Schnelltest muß auf jeden Fall der Arbeits-pH-Bereich beachtet werden. Man kann auch andere Gelatinen als Standard auswählen, sie müssen jedoch in ihrem IEP so gewählt werden, daß sie bei diesem pH-Wert entgegengesetzte Nettoladung tragen (siehe auch Fig.1). In den meisten Fällen werden die besagten Standardgelatinen Typ A und B für diesen Schnelltest ausreichen.

Ausgehend vom Ergebnis des Vorversuchs werden nun durch schrittweise Variation des IEP's durch Verwendung entsprechender Gelatinesorten und des pH-Wertes der Lösung in kleineren Bereichen (z. B. 0,1 pH-Schritte) die optimalen Bedingungen zur Bildung der Nanosole ermittelt. D.h. es muß das Stabilitätsoptimum, das durch den isoionischen Punkt (IIP) gekennzeichnet ist, gefunden werden, um eine ausreichende Stabilität für die genannten pharmazeutischen Anwendungen zu gewährleisten.

Es kann durchaus der Fall sein, daß eine im Sinne der Erfindung akzeptable Stabilität der Nanosole bereits in einem engeren pH-Bereich (ca. 0,5 Einheiten) um den isoionischen Punkt gefunden wird, so daß eine Einstellung dieses Punktes selbst nicht unbedingt notwendig ist. Andererseits können auch mehrere Gelatinen zu den gleichen, stabilen Ergebnissen führen. So kann beispielsweise (Beispiel 5) mit dem oralen Antidiabetikum Glibenclamid bei einem Gelatinetyp B mit einem IEP von 5,5 das Stabilitätsoptimum bei einem pH-Wert von 3,2 liegen, während bei einem Gelatinetyp B mit einem IEP von 3,8 das Stabilitätsoptimum bei einem pH-Wert von 2,2 liegt.

Gekennzeichnet durch ein Stabilitätsmaximum, wurde in beiden Fällen der isoionische Punkt erreicht (die Abhängigkeit der Nettoladung vom pH-Wert und dem IEP muß nicht linear sein, da sie durch den $pK_S$-Wert der vorhandenen COOH- bzw. $NH_3^+$ - Gruppen gegeben ist).

Erfindungsgemäß können auch andere makromolekulare Stoffe neben Gelatine, Kollagenhydrolysaten, fraktionierter Gelatine oder Gelatinederivaten in geringen Anteilen (maximal 5 Gew.-%) zugesetzt werden. Dabei kann es sich um amphotere bzw. geladene Stoffe, wie beispielsweise Albumine, Casein, Glykoproteine oder andere natürliche oder synthetische Polypeptide handeln. In besonderen Fällen können auch anionische Polymere wie z.B. Alginate, Gummi arabicum, Pektine, Polyacrylsäuren u.a. geeignet sein.

Es werden erfindungsgemäß mehrere Verfahren zur Herstellung der Nanosole vorgeschlagen. Dabei handelt es sich um eine beispielhafte, unvollständige Aufzählung. Der Fachmann kann aufgrund seines Fachwissens selbstständig weitere Varianten im Rahmen der vorliegenden Erfindung ausarbeiten:

## Verfahren I

Dieses kann angewendet werden, wenn der Arzneistoff in einer Mischung aus:
einem mit Wasser mischbaren organischen Lösungsmittel und Wasser, oder
aus mehreren mit Wasser mischbaren organischen Lösungsmitteln und Wasser
löslich ist:

a) eine in den Vorversuchen ausgewählte Gelatine wird mit Wasser in Solform überführt;

b) der in den Vorversuchen gefundene pH-Wert der Lösung wird eingestellt;

c) ein oder mehrere mit Wasser mischbare(s), organische(s) Lösungsmittel, vorzugsweise Ethanol, Isopropanol oder Methanol, wird/werden zu dieser Lösung gegeben;

d) der Arzneistoff wird in fester Form zu der Lösung gegeben und gelöst;

e) das/die organische(n) Lösungsmittel wird/werden entfernt, vorzugsweise durch Eindampfen in Vakuum; dabei entsteht das Nanosol;

f) die kolloid-disperse Lösung wird anschließend, vorzugsweise durch Sprüh- oder Gefriertrocknung, getrocknet.

Das organische Lösungsmittel hat die Aufgabe, den Arzneistoff zu lösen und verändert auch die Hydrathülle der Gelatinemoleküle.

<u>Verfahren II</u>

Diese Ausführungsform kann angewendet werden, wenn der Arzneistoff eine Säure oder eine Base ist, deren Salz in Wasser löslich ist:

a) eine in den Vorversuchen ausgewählte Gelatine wird mit $H_2O$ in die Solform überführt;

b) es wird ein solcher pH-Wert eingestellt, der die Salzbildung des Arzneistoffs ermöglicht;

c) der Arzneistoff wird unter Salzbildung in dem Gelatinesol gelöst;

d) durch Zugabe von Alkohol oder ähnlichen organischen Lösungsmitteln kann die Hydrathülle der Gelatinemoleküle gelockert werden;

e) durch Zugabe einer geeigneten Menge Säure oder Base wird der pH-Wert eingestellt, der zur Bildung des isoionischen Punkts (IIP) führt, dabei entsteht das Nanosol;

f) die kolloid-disperse Lösung wird wie in Verfahren I getrocknet.

Stufe d) ist fakultativ, jedoch bevorzugt

<u>Verfahren III</u>

Diese Ausführungsform kann angewendet werden, wenn der Arzneistoff ein Neutralstoff ist:

a) es wird ein Gelatinesol hergestellt, wie unter (1) a) und b) beschrieben.

b) eine zweite Lösung aus einem mit Wasser mischbaren organischen Lösungsmittel, vorzugsweise Ethanol, Methanol, Isopropanol, Aceton und dem Arzneistoff wird hergestellt.

c) die beiden Lösungen werden vereinigt.

d) das organische Lösungsmittel wird entfernt und die kolloid-disperse Lösung wird getrocknet.

<u>Verfahren IV</u>

a) Wie unter (I) a) und b) beschrieben.

b) In einer zweiten Lösung wird ein kolloid-disperses System mit dem Arzneistoff kurzzeitig gebildet, jedoch ohne Gelatine.

c) Die unter (b) erhaltene Lösung wird kontinuierlich mit der Gelatinelösung vereinigt.

Bei Schritt (IV) c) kann die kontinuierliche Vermischung der unter (IV) a) und b) beschriebenen Lösungen zeitabhängig durch on-line Messung der Teilchengröße mit einem geeigneten Verfahren, wie z.B. durch Laser-Licht-Streuung (BI-FOQELS On-line Particle Sizer), gesteuert werden. Damit ist es möglich, eine gewünschte Partikelgröße kontinuierlich einzustellen.

Alle genannten Verfahren sind auch für Kollagenhydrolysate und Gelatinederivate geeignet und können problemlos in den technischen Maßstab übertragen werden.

Die wesentlichen Schritte können weitgehend automatisiert ablaufen, wobei auch Verfahren I bis III kontinuierlich durchführbar sind.

Folgende Beispiele sollen die Erfindung näher erläutern:

**Beispiel 1**

| | |
|---|---|
| Arzneistoff: | Ibuprofen (Racemat), Wirkstoffsäure |

| | |
|---|---|
| Gelatinetyp: | handelsüblich, Typ B, 170 Bloom |
| Nanosol-Herstellung: | Analog Verfahren I |
| Gewichtsverhältnis Gelatine/Arzneistoff: | 1,5 : 1 |

Der Arbeits-pH-Bereich für Ibuprofen liegt vorzugsweise unterhalb seines $pK_s$-Wertes von 4,6.

Der Vortest bei pH 4,3 zur Ermittlung der Oberflächenladung der Ibuprofen-Partikel ergibt bei der Standardgelatine Typ B (IEP 3,5/200 Bloom) kein Nanosol. Unter gleichen Testbedingungen ergibt die Standardgelatine Typ A (IEP 9,5/200 Bloom) ein kurzzeitig stabiles Nanosol, bei dem die vorliegenden Ibuprofen-Partikel eine negative Oberflächenladung tragen.

Zur Ermittlung des Stabilitätsoptimums werden nun Gelatinesorten mit verschiedenen IEP's bei verschiedenen pH-Werten unterhalb pH 4,3 getestet. Die Meßreihe ergibt, daß sich eine Gelatine vom Typ B (IEP 4,9), die bei pH 3 eine positive Nettoladung trägt, am besten eignet. Das nach Verfahren I gebildete Nanosol weist ein für pharmazeutische Anwendung geeignetes Stabilitätsmaximum auf.

500 g einer 3%igen wäßrigen Gelatinelösung obengenannten Typs werden auf pH 3 gebracht.

250 ml Ethanol 96 % werden zugegeben.

10 g Ibuprofen werden in dieser Mischung gelöst, dann das organische Lösungsmittel evaporiert. Das so hergestellte Nanosol wird anschließend sprühgetrocknet und kann zur entsprechenden Arzneiform weiterverarbeitet werden.

Teilchengrößenmessungen mit einem BI-FOQELS On-line Particle Sizer ergeben zu 65 % Teilchengrößen von 450 nm.

**Beispiel 2**

Man verfährt wie unter Beispiel 1, verwendet jedoch eine Gelatine, die nach Beispiel III (Gelatineherstellung) mit gleichem Bloomwert und gleichem IEP (4,9) gewonnen wurde.

Teilchengrößenmessung ergeben zu 70% Teilchengrößen von 265 nm.

**Beispiel 3**

| | |
|---|---|
| Arzneistoff: | Dexamethason, Neutralstoff |
| Gelatinetyp: | Typ A, 220 Bloom, Herstellung Beispiel I |
| Nanosol-Herstellung: | Analog Verfahren III |
| Gewichtsverhältnis Gelatine/Arzneistoff: | ca. 10 : 1 |

Der bei pH 7 analog Beispiel 1 durchgeführte Vortest ergibt im Falle des Neutralstoffs Dexamethason, daß eine Gelatine Typ A geeignet ist.

Eine Gelatine Typ A (IEP 7,9) mit einem positiven Ladungszustand bei pH 5,3 ergibt das Stabilitätsoptimum.

500 g einer 7,5%igen wäßrigen Gelatinelösung aus oben spezifizierter Gelatinesorte wird durch Säurezusatz auf pH 5,3 eingestellt.

3,5 g Arzneistoff werden in 100 ml Aceton gelöst.

Beide Lösungen werden gemischt und das entstandene Nanosol nach Entfernung des organischen Lösungsmittels sprühgetrocknet.

Die durchschnittliche Teilchengröße des Nanosols liegt zwischen 260 und 300 nm.

**Beispiel 4**

Man stellt das Nanosol wie in Beispiel 3 her, aber unter Verwendung einer Gelatine handelsüblicher Qualität mit gleichen Kennzahlen.

Die durchschnittlichen Teilchengrößen liegen im Bereich von 550 bis 630 nm.

**Beispiel 5**

| | |
|---|---|
| Arzneistoff: | Glibenclamid, Wirkstoffsäure |
| Gelatinetyp: | Typ B, 100 Bloom, Herstellung Beispiel III |
| Nanosol-Herstellung: | Analog Verfahren III |
| Gewichtsverhältnis Gelatine/Arzneistoff: | 100 : 1 |

Der Arbeits-pH-Bereich für die schwache Wirkstoffsäure Glibenclamid liegt vorzugsweise unter ihrem $pK_s$-Wert von 6,3 bis 6,8.

Der erfindungsgemäße Vortest und die Meßreihe ergibt bei einem pH-Wert von 2,2 für den isoionischen Ladungszustand ein Optimum mit einer Gelatine Typ B (IEP 3,8).

Zur Nanosol-Herstellung werden nun 5 g Gelatine obigen Typs mit Wasser zu 5 % in die Solform überführt.

50 mg Glibenclamid werden in 30 ml Ethanol 96% gelöst und mit der wäßrigen Gelatinelösung homogen vermischt.

Das entstandene Nanosol wird nach Abrotieren des organischen Lösungsmittels lyophilisiert.

Durchschnittliche Teilchengrößen liegen bei 130 nm.

**Beispiel 6**

| | |
|---|---|
| Arzneistoff: | Propranolol, Wirkstoffbase |
| Gelatinetyp: | Typ B, 320 Bloom, Herstellung Beispiel II |
| Nanosol-Herstellung: | Analog Verfahren II |
| Gewichtsverhältnis Gelatine/Arzneistoff: | 4 : 1 |
| Arbeits-pH-Bereich: | 9,2 |
| Nach Vortest ausgewählte Gelatine: | Typ B/ 320 Bloom, IEP 4,2 |

In einer warmen Gelatinelösung (64 g und 640 ml Wasser) werden 16 g Propranolol-Hydrochlorid bei pH 3 aufgelöst. Durch Zugabe von Natronlauge wird ein pH-Wert von 8,8 eingestellt, bei dem sich ein Nanosol der Propranolol-Base bildet. In diesem Fall ist der isoionische Ladungszustand nur annähernd erreicht.

Die durchschnittlichen Teilchengrößen variieren stärker und liegen im Bereich von 650 bis 780 nm.

**Beispiel 7**

| | |
|---|---|
| Arzneistoff: | Indometacin, Wirkstoffsäure |
| Gelatinetyp: | Kollagenhydrolysat mit einem Peptidanteil von 90%, Herstellung Beispiel II |
| Nanosol-Herstellung: | Analog Verfahren II |
| Gewichtsverhältnis Gelatine/Arzneistoff: | 5 : 1 |

Der Vortest wird wie in Beispiel 1, jedoch bei einem bei einem pH-Wert von 4,0 durchgeführt.

Das Stabilitätsoptimum des Nanosols wird bei dem Kollagenhydrolysat mit einem IEP von 5,2 und einem pH-Wert der wäßrigen Arzneistoff/Gelatinelösung von 3,1 erreicht.

150 g des Kollagenhydrolysats werden in 2 l destilliertem Wasser aufgelöst. In dieser Lösung werden 30 g Indometacin suspendiert. Mit Natronlauge wird der pH-Wert des Systems zwischen 7 und 8 gehalten. Es wird solange weitergerührt, bis eine völlig klare Lösung entsteht. Danach wird durch Zugabe einer abgemessenen Menge Salzsäure der pH-Wert auf 3,1 eingestellt, bei dem sich spontan das Nanosol ausbildet,

Die erhaltene Nanosol-Lösung wird aufkonzentriert und sprühgetrocknet. Das erhaltene Pulver wird zu einer Akutform weiterverarbeitet.

Teilchengrößenmessung ergibt zu 70% Teilchengrößen kleiner als 370 nm.

**Beispiel 8**

| | |
|---|---|
| Arzneistoff: | Nifedipin, Neutralstoff |
| Gelatinetyp: | handelsüblich, Typ B, 60 Bloom |
| Nanosol-Herstellung: | analog Verfahren III |
| Gewichtsverhältnis Gelatine/Arzneistoff: | 15:1 |

Die Herstellung erfolgt unter Lichtschutz (Gelblicht).

Der Vortest wird analog Beispiel 1, jedoch bei einem pH-Wert von 6,0 durchgeführt.

Die anschließende Meßreihe zur Ermittlung des Stabilitätsoptimums ergibt eine Gelatine Typ B (IEP 4,7) bei einem pH-Wert von 5,5.

600 g oben spezifizierter, vollentsalzter Gelatine und 40 g PVP K 15 werden bei 40°C in 8 l destilliertem Wasser gelöst und auf einen pH-Wert von 5,5 eingestellt.

40 g Nifedipin werden in 1,3 l Ethanol vollständig gelöst.

Beide Lösungen werden homogen vermischt und das entstandene Nanosol nach Entfernung des Alkohols sprühgetrocknet. Das erhaltene Pulver wird in opake Hartgelatinekapseln mit einem Gehalt von 10 mg Nifedipin pro Kapsel abgefüllt.

Der Dissolutiontest (paddle) ergibt 100% Freisetzung nach 9 Minuten (75 Upm/ 900 ml 0,1 N HCl).

Die Bioverfügbarkeit wird in-vivo gegnüber herkömmlicher Kapselzubereitung mit mikronisiertem Nifedipin um 25% gesteigert. Maximale Blutspiegelwerte sind im Durchschnitt nach 20 Min. erreicht.

**Beispiel 9**

| | |
|---|---|
| Wirkstoff: | Glibenclamid, Wirkstoffsäure |
| Gelatinetyp: | handelsüblich, Typ B, Molekulargewicht unterhalb $10^4$ D |
| Nanosol-Herstellung: | analog Verfahren III |
| Gewichtsverhältnis Gelatine/Wirkstoff: | 35:1 |

Der Arbeits-pH-Bereich liegt unterhalb des $pK_S$-Wertes von 6,3-6,8.

Nach Durchführung des erfindungsgemäßen Vortests und der Meßreihe zur Bestimmung der optimalen Gelatinesorte wird ein Stabilitätsmaximum mit einer Gelatine Typ B (IEP 3,8) bei einem pH-Wert von 2,2 ermittelt.

500 g obiger Gelatine werden in 3 l dest. Wasser gelöst. Durch Zugabe von Salzsäure wird ein pH-Wert von 2,2 eingestellt.

13,89 g Glibenclamid werden in 0,2 l Ethanol gelöst. Die beiden Lösungen werden vereinigt, wobei sich das Nanosol bildet.

Teilchengrößenmessungen ergeben zu 80% Teilchengrößen kleiner 380 nm.

Das organische Lösungsmittel wird unter Vakuum entfernt und anschließend wird sprühgetrocknet.

Das getrocknete Nanosol wird unter Zusatz üblicher Tablettierhilfsstoffe auf einer Exzenterpresse zu Tabletten geformt. Es resultieren schnell anflutende Tabletten mit jeweils 3,5 mg Glibenclamidgehalt.

**Beispiel 10**:

| | |
|---|---|
| Wirkstoff: | Glibenclamid, Wirkstoffsäure |
| Gelatinetyp: | Typ B (IEP 3,8), 20 Bloom, Herstellung Beispiel III |
| Nanosol-Herstellung: | analog Verfahren III |
| Gewichtsverhältnis Gelatine/Wirkstoff: | 35:1 |

Die Herstellung des Nanosols erfolgt analog Beispiel 9.

Teilchengrößenmessungen ergeben zu 80% Teilchengrößen kleiner 180 nm.

**Beispiel 11**:

| | |
|---|---|
| Wirkstoff: | Indometacin, Wirkstoffsäure |
| Gelatinetyp: | handelsüblich, Typ B, 60 Bloom, |
| Nanosol-Herstellung: | analog Verfahren II |
| Gewichtsverhältnis Gelatine/Wirkstoff: | 6:1 |

Der Arbeits-pH-Bereich liegt unterhalb des $pK_S$-Wertes von 4,5.

Nach Durchführung des erfindungsgemäßen Vortests und der Meßreihe zur Bestimmung der optimalen Gelatinesorte wird ein Stabilitätsmaximum mit einer Gelatine Typ B (IEP 5,2) bei einem pH-Wert von 3,1 ermittelt.

600 g obiger Gelatine werden in 10 l dest. Wasser gelöst. In dieser Gelatinelösung werden 100 g Indometacin suspendiert. Natronlauge wird zugegeben, sodaß der pH-Wert des Systems im Bereich von 7 - 8 eingestellt wird. Es wird solange weitergerührt, bis eine klare Lösung entsteht. Danach wird durch Zugabe von Salzsäure auf pH 3,1 eingestellt, wobei sich das Nanosol bildet.

Durch anschließende Sprühtrocknung wird das Wasser entzogen. Unter Zusatz üblicher Tablettierhilfsstoffe wird das getrocknete Nanosol auf einer Exzenterpresse zu Tabletten verpreßt. Es resultieren schnell anflutende Tabletten mit jeweils 50 mg Indometacingehalt.

Teilchengrößenmessungen (BI-FOQUELS on-line Particle-Sizer) ergeben durchschnittliche Teilchengrößen von ca. 370 nm.

**Beispiel 12**:

Analog Beispiel 11, nur wird die Gelatine vor der Herstellung der Lösung mit 10 g Polyvinylpyrrolidon K 15 gemischt.

Teilchengrößenmessungen ergeben durchschnittliche Teilchengrößen von ca. 390 nm.

In einem Dissolutiontest nach USP (750 ml Prüfvolumen, bestehend aus 1 Volumenteil Phosphatpuffer pH 7,2 und 4 Volumenteilen Wasser, paddle, 100rpm, 37°C) ergibt sich eine vollständige Tablettenauflösung innerhalb von 15 Minuten. Im Vergleich dazu werden Tabletten aus Beispiel 11 unter gleichen Prüfbedingungen untersucht und zeigen im Durchschnitt um 20% höhere Auflösezeiten.

**Beispiel 13**:

| | |
|---|---|
| Wirkstoff: | Indometacin, Wirkstoffsäure |
| Gelatinetyp: | Kollagenhydrolysat (IEP 5,2), Herstellung nach Beispiel II |
| Nanosol-Herstellung: | analog Verfahren III |
| Gewichtsverhältnis | Gelatine/Wirkstoff: 4:1 |

300 g oben spezifizierter Gelatine werden in 3 l destilliertem Wasser gelöst und ein pH-Wert von 3,1 eingestellt.

75 g Indometacin werden in 500 ml Isopropanol gelöst. Beide Lösungen werden vereinigt und das organische Lösungsmittel durch Evaporation unter Vakuum entfernt, wobei sich das Nanosol bildet.

Anschließend wird lyophilisiert. Das erhaltene Pulver wird in opake Hartgelatinekapseln mit einem Gehalt von jeweils 25 mg Indometacin abgefüllt.

Diese Kapseln ergeben in einem Dissolutiontest nach USP (750 ml Prüfvolumen, bestehend aus 1 Volumenteil Phosphatpuffer pH 7,2 und 4 Volumenteilen Wasser, Drehkörbchen, 100 rpm, 37°C) einen Kapselzerfall innerhalb von 3 min.

**Patentansprüche**

1. Verfahren zur Herstellung eines kolloid-dispersen Systems von in Wasser schwerlöslichen anorganischen und/oder organischen Verbindungen, dadurch gekennzeichnet, daß man

    a) eine Gelatine, ein Koallagenhydrolysat oder ein Gelatinederivat nach ihrem (seinem) isoelektrischen Punkt (IEP) so auswählt, daß ihr (sein) IEP mit dem Ladungszustand der Partikel der anorganischen und/oder organischen Verbindung so abgestimmt ist, daß die Gelatine, das Kollagenhydrolysat oder das Gelatinederivat bei einem bestimmten pH-Wert mit der ungelösten anorganischen und/oder organischen Verbindung(en) zu Ladungsneutralität führt,

    b) die Gelatine, das Kollagenhydrolysat oder das Gelatinederivat in die wäßrige Solform überführt,

    c) den pH-Wert in Abhängigkeit von dem IEP der Gelatine Kollagenhydrolysat oder Gelatinederivat auf einen solchen Wert einstellt, daß die sich bildenden Nanopartikel der anorganischen und/oder organischen Verbindung nahezu oder vollständig ladungsneutral stabilisiert werden, und

    d) vor oder nach Stufe c) die anorganische und/oder organische Verbindung in dem wäßrigen Sol von Gelatine, Kollagenhydrolysat oder Gelatinederivat löst oder eine Lösung der anorganischen und/oder organischen Verbindung mit dem wäßrigen Gelatinesol vereinigt.

2. Verfahren nach Anspruch 1, dadurch gekennzeichnet, daß man in Stufe d) die gelöste anorganische und/oder organische Verbindung vor der Vereinigung mit dem wäßrigen Sol von Gelatine, Kollagenhydrolysat oder Gelatinederivat kurzzeitig in kolloid-disperse Form von Nanopartikel überführt und die so erhaltene Dispersion von Nanopartikeln kontinuierlich mit dem wäßrigen Gelatinesol vereinigt.

3. Verfahren nach Anspruch 1 oder 2, dadurch gekennzeichnet, daß man

    e1) die anorganische und/oder organische Verbindung in Form von Nanopartikeln ausfällt.

4. Verfahren nach Anspruch 1, dadurch gekennzeichnet, daß man als anorganische und/oder organische Verbindung einen in Wasser schwer löslichen Arzneistoff einsetzt.

5. Verfahren nach einem der Ansprüche 1 bis 4, dadurch gekennzeichnet, daß man

    e2) die in Stufe d) erhaltene kolloid-disperse Lösung sprühtrocknet oder gefriertrocknet und so ein stabiles resuspendierbares Nanosol erhält, das nach Wiederauflösung in wäßrigem Medium ein kolloid-disperses System in Nanosolform ergibt.

6. Verfahren nach einem der Ansprüche 1 bis 5, dadurch gekennzeichnet, daß man in Stufe d) den Arzneistoff in einem mit Wasser mischbaren organischen Lösungsmittel gelöst, zusetzt.

7. Verfahren nach einem der Ansprüche 1 bis 6, dadurch gekennzeichnet, daß man den Arzneistoff, der eine Säure oder Base ist, in sein Salz überführt.

8. Verfahren nach einem der Ansprüche 1 bis 6, dadurch gekennzeichnet, daß man die mit Wasser mischbaren organischen Lösungsmittel in Stufe b) dem wäßrigen Gelatinesol zusetzt und
in Stufe d) den Arzneistoff in fester Form dieser Mischung zusetzt und damit löst.

9. Verfahren nach Anspruch 6 oder 8, dadurch gekennzeichnet, daß man das organische Lösungsmittel anschließend wieder entfernt.

10. Verfahren nach einem der Ansprüche 1 bis 4 und/oder 9 dadurch gekennzeichnet, daß man die Nanopartikel-Lösung anschließend trocknet.

11. Verfahren nach Anspruch 9, dadurch gekennzeichnet, daß man die Lösung sprühtrocknet oder gefriertrocknet.

12. Verfahren nach einem der Ansprüche 1 bis 4, dadurch gekennzeichnet, daß man vor Einstellung des pH-Wertes auf den isoionischen Punkt in Stufe c) den pH-Wert so einstellt, daß der Arzneistoff ein Salz bildet.

13. Verfahren nach einem der Ansprüche 1 bis 12, dadurch gekennzeichnet, daß man im Anschluß an Stufe d) ein mit Wasser mischbares organisches Lösungsmittel zur Lockerung der Hydrathülle der Gelatinemoleküle zusetzt.

14. Verfahren nach einem der Ansprüche 1 bis 12, dadurch gekennzeichnet, daß man in Stufe b) zusätzlich Polyvinylpyrrolidon in einem Gewichtsverhältnis von Gelatine zu Polyvinylpyrrolidon wie 5:1 bis 500:1 vorzugsweise 5:1 bis 30:1 zugibt.

15. Verfahren nach Anspruch 13, dadurch gekennzeichnet, daß man einen Alkohol zusetzt.

16. Verfahren nach einem der Ansprüche 1 bis 15, dadurch gekennzeichnet, daß man die kolloiden Teilchen kontinuierlich mit einer einstellbaren Partikelgröße herstellt.

17. Verfahren nach Anspruch 16, dadurch gekennzeichnet, daß man die Teilchengröße kontinuierlich mißt.

18. Verfahren nach einem der Ansprüche 1 bis 17, dadurch gekennzeichnet, daß man es kontinuierlich durchführt.

19. Verfahren nach einem der Ansprüche 1 bis 18, dadurch gekennzeichnet, daß man eine Gelatine verwendet, bei der der native Zustand des Kollagens weitgehend erhalten ist.

20. Verfahren nach Anspruch 19, dadurch gekennzeichnet, daß man eine Gelatine mit kurzer Erstarrungszeit (Peptidanteil < 20%) einsetzt.

21. Verfahren nach einem der Ansprüche 1 bis 20, dadurch gekennzeichnet, daß man eine Gelatine vom Typ A einsetzt.

22. Verfahren nach einem der Ansprüche 1 bis 20, dadurch gekennzeichnet, daß man eine Gelatine vom Typ B einsetzt.

23. Verfahren nach einem der Ansprüche 1-22, dadurch gekennzeichnet, daß man als Arzneistoff Glibenclamid einsetzt.

24. Verfahren nach einem der Ansprüche 1-22, dadurch gekennzeichnet, daß man als Arzneistoff ein 3-Indolylessigsäurederivat einsetzt.

25. Nanosol von in Wasser schwer löslichen anorganischen und/oder organischen Verbindungen oder Gemischen von anorganischen und/oder organischen Verbindungen mit Gelatine, gekennzeichnet durch

a) eine innere Phase aus der oder den anorganischen und/oder organischen Verbindung(en), die eine Teilchengröße von 10 - 800 nm aufweist (aufweisen) und eine negative oder positive Oberflächenladung besitzt (besitzen),

b) eine äußere Phase aus Gelatine, einem Kollagenhydrolysat oder einem Gelatinederivat, welche(s) positiv oder negativ geladen ist,

c) einen annähernd oder vollständig isoionischen Ladungszustand der inneren und äußeren Phase.

**26.** Nanosol nach Anspruch 25, dadurch gekennzeichnet, daß die anorganische(n) und/oder organische(n) Verbindung(en) eine Partikelgrößenverteilung unterhalb 400 nm, insbesondere 10 - 100 nm aufweist (aufweisen).

**27.** Nanosol nach Anspruch 25 und/oder 26, dadurch gekennzeichnet, daß bei positiv geladenen Teilchen die Gelatine eine negative Ladung oberhalb einem pH-Wert von 3,5 in wäßriger Lösung aufweist.

**28.** Nanosol nach einem der Ansprüche 25 bis 27, dadurch gekennzeichnet, daß bei negativ geladenen Teilchen die Gelatine eine positive Ladung unterhalb von einem pH-Wert von 9,5 in wäßriger Lösung aufweist.

**29.** Nanosol nach einem der Ansprüche 25 bis 27, dadurch gekennzeichnet, daß die anorganische und/oder organische Verbindung ein in Wasser schwer löslicher Arzneistoff und das Nanosol pharmazeutisch applizierbar ist.

**30.** Nanosol nach Anspruch 25, dadurch gekennzeichnet, daß es als flüssige, wäßrige Nanodispersion vorliegt.

**31.** Nanosol nach Anspruch 25, dadurch gekennzeichnet, daß es als feste, resuspendierbare Nanodispersion vorliegt.

**32.** Nanosol nach einem der Ansprüche 25 bis 31, gekennzeichnet durch eine äußere Phase, die zusätzlich Polyvinylpyrrolidon in einem Gewichtsverhältnis von Gelatine zu Polyvinylpyrrolidon wie 5:1 bis 500:1 insbesondere 5:1 bis 30:1 enthält.

**33.** Nanosol nach einem der Ansprüche 29 bis 32 dadurch gekennzeichnet, daß der Arzneistoff eine Löslichkeit in Wasser bei Raumtemperatur von kleiner als 5 g/l, insbesondere kleiner als 1 g/l aufweist.

**34.** Nanosol nach Anspruch 29, dadurch gekennzeichnet, daß die pharmazeutisch applizierbare Form eine Tablette ist, die den Arzneistoff schnell freisetzt.

**35.** Nanosol nach Anspruch 29, dadurch gekennzeichnet, daß die pharmazeutisch applizierbare Form eine Tablette ist, die den Arzneistoff langsam freisetzt.

**36.** Nanosol nach Anspruch 29, dadurch gekennzeichnet, daß die pharmazeutisch applizierbare Form eine feste, pulverförmige, in Hartgelatinekapseln abgefüllte Nanodispersion darstellt.

**37.** Nanosol nach Anspruch 29, dadurch gekennzeichnet, daß die pharmazeutisch applizierbare Form eine halbfeste Arzneiform darstellt.

**38.** Nanosol nach Anspruch 29, dadurch gekennzeichnet, daß die pharmazeutisch applizierbare Form eine parenterale Arzneiform darstellt.

**39.** Nanosol nach Anspruch 29, dadurch gekennzeichnet, daß die pharmazeutisch applizierbare Form eine bioadhäsive Arzneiform darstellt.

**40.** Akut-Arzneimittel zur Behandlung von Diabetes, enthaltend Glibenclamid neben üblichen pharmazeutischen Trägern und Hilfsstoffen, dadurch gekennzeichnet, daß das Glibenclamid in Form eines pharmazeutisch applizierbaren Nanosols nach einem der Ansprüche 29-39 vorliegt.

**41.** Akut-Arzneimittel zur Behandlung von rheumatischen und/oder entzündlichen Erkrankungen, enthaltend ein 3-Indolylessigsäurederivat neben üblichen pharmazeutischen Trägern und Hilfsstoffen, dadurch gekennzeichnet, daß das 3-Indolylessigsäurederivat in Form eines pharmazeutisch applizierbaren Nanosols nach einem der Ansprüche 29-39 vorliegt.

**42.** Arzneimittel nach Anspruch 40 und/oder 41, dadurch gekennzeichnet, daß die Gelatine ein Maximum der Molekulargewichtsverteilung unterhalb von $10^5$ D und Bloomwerte von 0 - 240 aufweist.

**43.** Arzneimittel nach einem der Ansprüche 40 bis 42, dadurch gekennzeichnet, daß die Gelatine einen Bloomwert von 0 - 170 aufweist.

**44.** Arzneimittel nach Anspruch 43, dadurch gekennzeichnet, daß die Gelatine einen Peptidanteil von 50 - 90% aufweist.

**45.** Arzneimittel nach einem der Ansprüche 40 bis 44, dadurch gekennzeichnet, daß die Gelatine ein Maximum der Molekulargewichtsverteilung im Bereich von $10^4$ - 9,5 x $10^4$ D aufweist.

**46.** Arzneimittel nach einem der Ansprüche 40 bis 45, dadurch gekennzeichnet, daß das Gewichtsverhältnis Gelatine zu Wirkstoff 1:1 bis 200:1 ist.

**47.** Arzneimittel nach einem der Ansprüche 40 bis 46, dadurch gekennzeichnet, daß die Arzneiform ein schnell auflösendes Pulver oder Granulat ist.

**48.** Arzneimittel nach einem der Ansprüche 40-47, dadurch gekennzeichnet, daß es teilweise als Akutform, teilweise als Retardform vorliegt.

**49.** Arzneimittel nach Anspruch 41 und/oder 48, dadurch gekennzeichnet, daß das 3-Indolylessigsäurederivat Indometacin ist.

**50.** Arzneimittel nach Anspruch 41 und/oder 48, dadurch gekennzeichnet, daß das 3-Indolylessigsäurederivat Acemetacin ist.

**51.** Arzneimittel nach einem der Ansprüche 40 bis 50, dadurch gekennzeichnet, daß die Gelatine einen Anteil an rechtsdrehenden Aminosäuren unterhalb von 20% aufweist.

**52.** Arzneimittel nach einem der Ansprüche 40 bis 51, dadurch gekennzeichnet, daß die Arzneiform eine Hartgelatinekapsel mit schnell auflösendem Pulver und langsam auflösender Tablette ist.

## Claims

**1.** Process for the preparation of a colloidally disperse system of poorly water-soluble inorganic and/or organic compounds, characterized in that

a) a gelatin, a collagen hydrolysate or a gelatin derivative is selected according to its isoelectric point (IEP) such that its IEP is suited to the charge state of the particles of the inorganic and/or organic compound such that the gelatin, the collagen hydrolysate or the gelatin derivative leads to charge neutrality with the undissolved organic compound at a specific pH,
b) the gelatin, the collagen hydrolysate or the gelatin derivative is converted into the aqueous sol form,
c) the pH is adjusted as a function of the IEP of the gelatin to a value such that the nanoparticles of the inorganic and/or organic compound which form are nearly or completely stabilized in a neutrally charged manner, and
d) before or after stage c) the inorganic and/or organic compound is dissolved in the aqueous gelatin sol or a solution of the inorganic and/or organic compound is combined with the aqueous gelatin sol.

**2.** Process according to Claim 1, characterized in that in stage d) the dissolved inorganic and/or organic compound is converted into a colloidally disperse form of nanoparticles briefly before combination with the aqueous gelatin sol and the dispersion of nanoparticles thus obtained is continuously combined with the aqueous gelatin sol.

**3.** Process according to Claim 1 or 2, characterized in that

e1) the inorganic and/or organic compound is precipitated in the form of nanoparticles.

**4.** Process according to Claim 1, characterized in that a poorly water-soluble pharmaceutical substance is employed as the inorganic and/or organic compound.

**5.** Process according to one of Claims 1 to 4, characterized in that

e2) the colloidally disperse solution obtained in stage d) is spray-dried or freeze-dried and a stable resuspensible nanosol is thus obtained, which after redissolution in aqueous medium yields a colloidally disperse system in nanosol form.

**6.** Process according to one of Claims 1 to 5, characterized in that the pharmaceutical substance dissolved in a water-miscible organic solvent is added in stage d).

7. Process according to one of Claims 1 to 6, characterized in that the pharmaceutical substance, which is an acid or base, is converted into its salt.

8. Process according to one of Claims 1 to 6, characterized in that the water-miscible organic solvent is added to the aqueous gelatin sol in stage b) and the pharmaceutical substance is added to this mixture in stage d) and thus dissolved.

9. Process according to Claim 6 or 8, characterized in that the organic solvent is then removed again.

10. Process according to one of Claims 1 to 4 and/or 9, characterized in that, the nanoparticle solution is then dried.

11. Process according to Claim 9, characterized in that the solution is spray-dried or freeze-dried.

12. Process according to one of Claims 1 to 4, characterized in that, before adjusting the pH to the isoionic point in stage c), the pH is adjusted such that the pharmaceutical substance forms a salt.

13. Process according to one of Claims 1 to 12, characterized in that following stage d) a water-miscible organic solvent is added to loosen the hydration shell of the gelatin molecules.

14. Process according to one of Claims 1 to 12, characterized in that polyvinylpyrrolidone in a weight ratio of gelatin to polyvinylpyrrolidone such as 5 : 1 to 500 : 1, preferably 5 : 1 to 30 : 1, is additionally added in stage b).

15. Process according to Claim 13, characterized in that an alcohol is added.

16. Process according to one of Claims 1 to 15, characterized in that the colloidal particles are prepared continuously with an adjustable particle size.

17. Process according to Claim 16, characterized in that the particle size is continuously measured.

18. Process according to one of Claims 1 to 17, which process is carried out continuously.

19. Process according to one of Claims 1 to 18, characterized in that a gelatin is used in which the native state of the collagen is largely maintained.

20. Process according to Claim 19, characterized in that a gelatin having a short solidification time (peptide content < 20 %) is employed.

21. Process according to one of Claims 1 to 20, characterized in that a gelatin of the type A is employed.

22. Process according to one of Claims 1 to 20, characterized in that a gelatin of the type B is employed.

23. Process according to one of Claims 1 - 22, characterized in that glibenclamide is employed as the pharmaceutical substance.

24. Process according to one of Claims 1 - 22, characterized in that a 3-indolylacetic acid derivative is employed as the pharmaceutical substance.

25. Nanosol of poorly water-soluble inorganic and/or organic compounds or mixtures of inorganic and/or organic compounds with gelatin, characterized by

a) an inner phase of the organic compound(s), which has (have) a particle size of 10 - 800 nm and possess(es) a negative or positive surface charge,
b) an outer phase of gelatin, a collagen hydrolysate or a gelatin derivative, which is oppositely (positively or negatively) charged,
c) an approximately or completely isoionic charge state of the inner and outer phase.

26. Nanosol according to Claim 25, characterized in that the inorganic and/or organic compound(s) has (have) a particle size distribution of below 400 nm, in particular 10 - 100 nm.

27. Nanosol according to Claims 25 and/or 26, characterized in that with positively charged particles the gelatin has a negative charge above a pH of 3.5 in aqueous solution.

28. Nanosol according to one of Claims 25 to 27, characterized in that with negatively charged particles the gelatin has a positive charge below a pH of 9.5 in aqueous solution.

29. Nanosol according to one of Claims 25 to 27, characterized in that the inorganic and/or organic compound is poorly water-soluble pharmaceutical substance and the nanosol is pharmaceutically administrable.

30. Nanosol according to Claim 25, which is present as a liquid, aqueous nanodispersion.

31. Nanosol according to Claim 25, which is present as a solid, resuspensible nanodispersion.

32. Nanosol according to one of Claims 25 to 31, characterized by an outer phase which additionally contains polyvinylpyrrolidone in a weight ratio of gelatin to polyvinylpyrrolidone such as 5 : 1 to 500 : 1, in particular 5 : 1 to 30 : 1.

33. Nanosol according to one of Claims 29 to 32, characterized in that the pharmaceutical substance has a solubility in water at room temperature of less than 5 g/l, in particular less than 1 g/l.

34. Nanosol according to Claim 29, characterized in that the pharmaceutically administrable form is a tablet which rapidly releases the pharmaceutical substance.

35. Nanosol according to Claim 29, characterized in that the pharmaceutically administrable form is a tablet which slowly releases the pharmaceutical substance.

36. Nanosol according to Claim 29, characterized in that the pharmaceutically administrable form is a solid, powdered nanodispersion filled into hard gelatin capsules.

37. Nanosol according to Claim 29, characterized in that the pharmaceutically administrable form is a semisolid pharmaceutical form.

38. Nanosol according to Claim 29, characterized in that the pharmaceutically administrable form is a parenteral pharmaceutical form.

39. Nanosol according to Claim 29, characterized in that the pharmaceutically administrable form is a bioadhesive pharmaceutical form.

40. Immediate-effect medicament for the treatment of diabetes, containing glibenclamide in addition to customary pharmaceutical excipients and auxiliaries, characterized in that the glibenclamide is present in the form of a pharmaceutically administrable nanosol according to one of Claim 29 - 39.

41. Immediate-effect medicament for the treatment of rheumatic and/or inflammatory disorders, containing a 3-indolylacetic acid derivative in addition to customary pharmaceutical excipients and auxiliaries, characterized in that the 3-indolylacetic acid derivative is present in the form of a pharmaceutically administrable nanosol according to one of Claims 29 - 39.

42. Medicament according to Claims 40 and/or 41, characterized in that the gelatin has a maximum in the molecular weight distribution of below $10^5$ D and bloom values of 0 - 240.

43. Medicament according to one of Claims 40 to 42, characterized in that the gelatin has a bloom value of 0 - 170.

44. Medicament according to Claim 43, characterized in that the gelatin has a peptide content of 50 - 90 %.

45. Medicament according to one of Claims 40 to 44, characterized in that the gelatin has a maximum in the molecular weight distribution in the range $10^4$ - $9.5 \times 10^4$ D.

46. Medicament according to one of Claims 40 to 45, characterized in that the weight ratio gelatin to active compound is 1 : 1 to 200 : 1.

47. Medicament according to one of Claims 40 to 46, characterized in that the pharmaceutical form is a rapidly dissolving powder or granules.

48. Medicament according to one of Claims 40 - 47, characterized in that it is partly present as an immediate-effect form and partly as a sustained-release form.

49. Medicament according to Claims 41 and/or 48, characterized in that the 3-indolylacetic acid derivative is indometacin.

50. Medicament according to Claims 41 and/or 48, characterized in that the 3-indolylacetic acid derivative is acemetacin.

51. Medicament according to one of claims 40 to 50, characterized in that the gelatin has a content of dextrorotatory amino acids of below 20 %.

52. Medicament according to one of Claims 40 to 51, characterized in that the pharmaceutical form is a hard gelatin capsule containing rapidly dissolving powder and a slowly dissolving tablet.

## Revendications

1. Procédé de préparation d'un système en dispersion colloïdale de composés minéraux et/ou organiques peu solubles dans l'eau, caractérisé en ce que :

   a) on choisit une gélatine, un hydrolysat de collagène ou un dérivé de la gélatine en fonction de leur point isoélectrique (PIE) en accordant leur PIE à l'état de charge des particules du composé minéral et/ou organique de telle sorte que la gélatine, l'hydrolysat de collagène ou le dérivé de la gélatine, à un pH déterminé, conduise avec le composé organique non dissous à la neutralité de la charge ;
   b) on amène la gélatine, l'hydrolysat de collagène ou le dérivé de la gélatine sous la forme d'un sol aqueux ;
   c) on ajuste le pH en fonction du PIE de la gélatine à une valeur telle que les nanoparticules du composé minéral et/ou organique qui se forment soient à peu près ou complètement stabilisées à la neutralité de la charge ;
   d) avant ou après l'étape c), on dissout le composé minéral et/ou organique dans le sol aqueux de gélatine ou on réunit une solution du composé minéral et/ou organique et le sol aqueux de gélatine.

2. Procédé selon la revendication 1, caractérisé en ce que dans l'étape d), on transforme temporairement le composé minéral et/ou organique dissous, avant la réunion avec le sol aqueux de gélatine, en la forme en dispersion colloïdale de nanoparticules, et on réunit en continu la dispersion de nanoparticules ainsi obtenue et le sol aqueux de gélatine.

3. Procédé selon la revendication 1 ou 2, caractérisé en ce que

   e1) on précipite le composé minéral et/ou organique sous forme de nanoparticules.

4. Procédé selon la revendication 1, caractérisé en ce qu'on utilise comme composé minéral et/ou organique un médicament peu soluble dans l'eau.

5. Procédé selon l'une des revendications 1 à 4, caractérisé en ce que

   e2) on sèche par pulvérisation ou on lyophilise la solution en dispersion colloïdale obtenue dans l'étape d) et l'on obtient ainsi un nanosol stable pouvant être remis en suspension qui, après redissolution dans un milieu aqueux, donne un système en dispersion colloïdale sous forme de nanosol.

6. Procédé selon l'une des revendications 1 à 5, caractérisé en ce que dans l'étape d), on ajoute le médicament dissous dans un solvant organique miscible à l'eau.

7. Procédé selon l'une des revendications 1 à 6, caractérisé en ce que l'on transforme le médicament, qui est un acide ou une base, en son sel.

8. Procédé selon l'une des revendications 1 à 6, caractérisé en ce que dans l'étape b) on ajoute les solvants organiques miscibles à l'eau au sol aqueux de gélatine, et

   dans l'étape d), on ajoute le médicament sous forme solide à ce mélange et on le dissout avec celui-ci.

9. Procédé selon la revendication 6 ou 8, caractérisé en ce qu'on élimine ensuite à nouveau le solvant organique.

10. Procédé selon l'une des revendications 1 à 4 et/ou 9, caractérisé en ce que l'on sèche ensuite la solution de nanoparticules.

11. Procédé selon la revendication 9, caractérisé en ce que l'on sèche la solution par pulvérisation ou par lyophilisation.

12. Procédé selon l'une des revendications 1 à 4, caractérisé en ce qu'avant l'ajustement du pH au point isoionique dans l'étape c), on ajuste le pH de telle sorte que le médicament forme un sel.

13. Procédé selon l'une des revendications 1 à 12, caractérisé en ce qu'à la suite de l'étape d), on ajoute un solvant organique miscible à l'eau pour relâcher l'enveloppe d'hydrate des molécules de gélatine.

14. Procédé selon l'une des revendications 1 à 12, caractérisé en ce que dans l'étape b), on ajoute en outre de la polyvinylpyrrolidone dans un rapport pondéral de la gélatine à la polyvinylpyrrolidone de 5:1 à 500:1, de préférence de 5:1 à 30:1.

15. Procédé selon la revendication 13, caractérisé en ce que l'on ajoute un alcool.

16. Procédé selon l'une des revendications 1 à 15, caractérisé en ce que l'on prépare en continu les particules colloïdales avec une grandeur réglable.

17. Procédé selon la revendication 16, caractérisé en ce que l'on mesure la grandeur des particules en continu.

18. Procédé selon l'une des revendications 1 à 17, caractérisé en ce qu'on le met en oeuvre en continu.

19. Procédé selon l'une des revendications 1 à 18, caractérisé en ce que l'on utilise une gélatine dans laquelle l'état naturel du collagène est conservé dans une large mesure.

20. Procédé selon la revendication 19, caractérisé en ce que l'on utilise une gélatine ayant un court temps de solidification (proportion de peptides < 20 %).

21. Procédé selon l'une des revendications 1 à 20, caractérisé en ce que l'on utilise une gélatine de type A.

22. Procédé selon l'une des revendications 1 à 20, caractérisé en ce que l'on utilise une gélatine de type B.

23. Procédé selon l'une des revendications 1 à 22, caractérisé en ce que l'on utilise comme médicament le glibenclamide.

24. Procédé selon l'une des revendications 1 à 22, caractérisé en ce que l'on utilise comme médicament un dérivé de l'acide 3-indolylacétique.

25. Nanosol de composés minéraux et/ou organiques ou de mélanges de composés minéraux et/ou organiques peu solubles dans l'eau avec de la gélatine, caractérisé par

   a) une phase interne constituée du ou des composés organiques, qui présente(nt) une grandeur de particules de 10 à 800 nm et possède(nt) une charge superficielle négative ou positive,
   b) une phase externe de gélatine, d'un hydrolysat de collagène ou d'un dérivé de gélatine qui est chargé positivement ou négativement,
   c) un état de charge des phases interne et externe à peu près ou complètement isoionique.

26. Nanosol selon la revendication 25, caractérisé en ce que le ou les composés minéraux et/ou organiques présentent une répartition des grandeurs de particules au-dessous de 400 nm, en particulier de 10 à 100 nm.

27. Nanosol selon les revendications 25 et/ou 26, caractérisé en ce que dans le cas de particules chargées positivement, la gélatine présente une charge négative audessus d'un pH de 3,5 en solution aqueuse.

28. Nanosol selon l'une des revendications 25 à 27, caractérisé en ce que dans le cas de particules chargées négativement, la gélatine présente une charge positive au-dessous d'un pH de 9,5 en solution aqueuse.

**29.** Nanosol selon l'une des revendications 25 à 27, caractérisé en ce que le composé minéral et/ou organique est un médicament peu soluble dans l'eau et en ce que le nanosol est administrable pharmaceutiquement.

**30.** Nanosol selon la revendication 25, caractérisé en ce qu'il se présente sous la forme d'une nanodispersion liquide, aqueuse.

**31.** Nanosol selon la revendication 25, caractérisé en ce qu'il se présente sous la forme d'une nanodispersion solide pouvant être remise en suspension.

**32.** Nanosol selon l'une des revendications 25 à 31, caractérisé par une phase externe qui contient en outre de la polyvinylpyrrolidone dans un rapport pondéral de la gélatine à la polyvinylpyrrolidone de 5:1 à 500:1, en particulier de 5:1 à 30:1.

**33.** Nanosol selon l'une des revendications 29 à 32, caractérisé en ce que le médicament présente une solubilité dans l'eau à la température ambiante inférieure à 5 g/l, en particulier inférieure à 1 g/l.

**34.** Nanosol selon la revendication 29, caractérisé en ce que la forme pharmaceutiquement administrable est un comprimé qui libère rapidement le médicament.

**35.** Nanosol selon la revendication 29, caractérisé en ce que la forme pharmaceutiquement administrable est un comprimé qui libère lentement le médicament.

**36.** Nanosol selon la revendication 29, caractérisé en ce que la forme pharmaceutiquement administrable représente une nanodispersion solide en poudre chargée dans des capsules de gélatine dure.

**37.** Nanosol selon la revendication 29, caractérisé en ce que la forme pharmaceutiquement administrable est une forme médicamenteuse semi-solide.

**38.** Nanosol selon la revendication 29, caractérisé en ce que la forme pharmaceutiquement administrable est une forme médicamenteuse parentérale.

**39.** Nanosol selon la revendication 29, caractérisé en ce que la forme pharmaceutiquement administrable est une forme médicamenteuse bio-adhésive.

**40.** Médicament à libération rapide pour le traitement du diabète, contenant du glibenclamide en plus de supports et d'adjuvants pharmaceutiques habituels, caractérisé en ce que le glibenclamide est présent sous la forme d'un nanosol pharmaceutiquement administrable selon l'une des revendications 29 à 39.

**41.** Médicament à libération rapide pour le traitement d'affections rhumatismales et/ou inflammatoires, contenant un dérivé de l'acide 3-indolylacétique en plus de supports et d'adjuvants pharmaceutiques habituels, caractérisé en ce que le dérivé de l'acide 3-indolylacétique est présent sous la forme d'un nanosol pharmaceutiquement administrable selon l'une des revendications 29 à 39.

**42.** Médicament selon les revendications 40 et/ou 41, caractérisé en ce que la gélatine présente un maximum de la répartition des masses moléculaires inférieur à $10^5$ D et des indices Bloom de 0 à 240.

**43.** Médicament selon l'une des revendications 40 à 42, caractérisé en ce que la gélatine a un indice Bloom de 0 à 170.

**44.** Médicament selon la revendication 43, caractérisé en ce que la gélatine présente une proportion de peptides de 50 à 90 %.

**45.** Médicament selon l'une des revendications 40 à 44, caractérisé en ce que la gélatine présente un maximum de la répartition des masses moléculaires dans la plage de $10^4$ à $9,5 \times 10^4$ D.

**46.** Médicament selon l'une des revendications 40 à 45, caractérisé en ce que le rapport pondéral de la gélatine à la substance active est de 1:1 à 200:1.

**47.** Médicament selon l'une des revendications 40 à 46, caractérisé en ce que la forme médicamenteuse est une poudre ou un granulé se dissolvant rapidement.

**48.** Médicament selon l'une des revendica-tions 40 à 47, caractérisé en ce qu'il est présent en partie sous une forme à libération rapide, et en partie sous forme à libération retardée.

**49.** Médicament selon les revendications 41 et/ou 48, caractérisé en ce que le dérivé de l'acide 3-indolylacétique est l'indométacine.

**50.** Médicament selon les revendications 41 et/ou 48, caractérisé en ce que le dérivé de l'acide 3-indolylacétique est l'acémétacine.

**51.** Médicament selon l'une des revendications 40 à 50, caractérisé en ce que la gélatine présente une proportion d'aminoacides dextrogyres inférieure à 20 %.

**52.** Médicament selon l'une des revendications 40 à 51, caractérisé en ce que la présentation médicamenteuse est une capsule de gélatine dure avec une poudre se dissolvant rapidement et un comprimé se dissolvant lentement.

Fig. 1

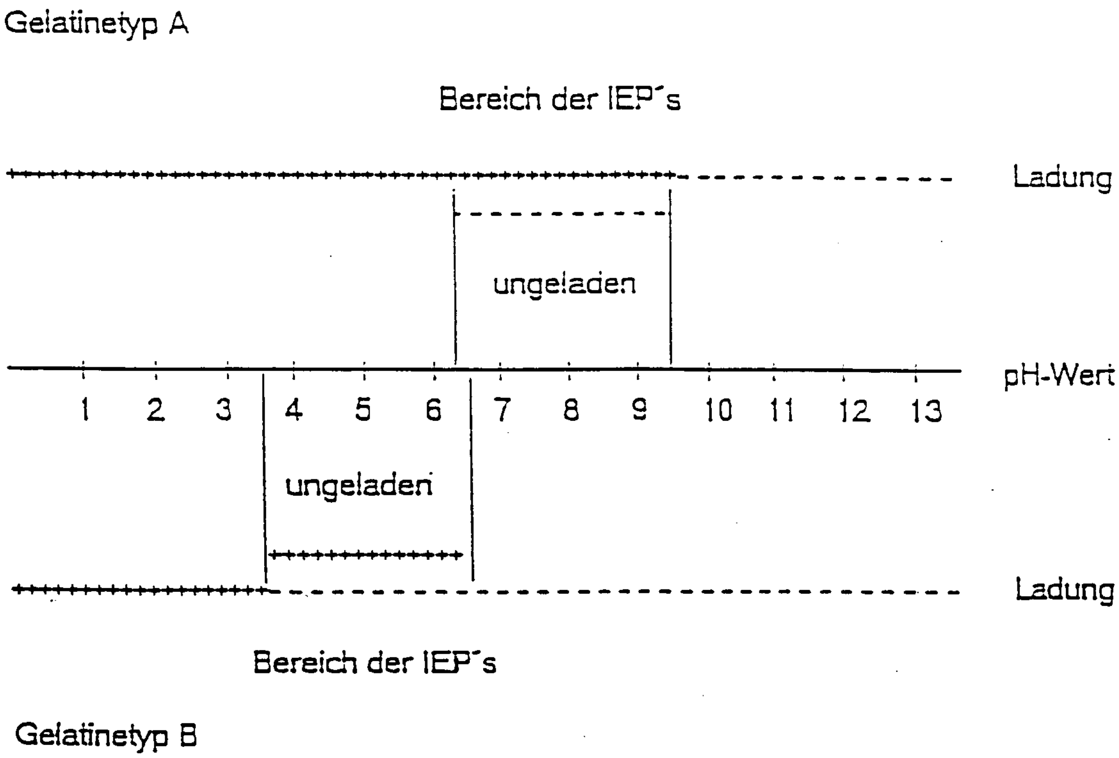


Ladungsverteilungen in den Gelatinetypen A (sauer) und B (alkalisch)

IEP = isoelektrischer Punkt

Fig. 2

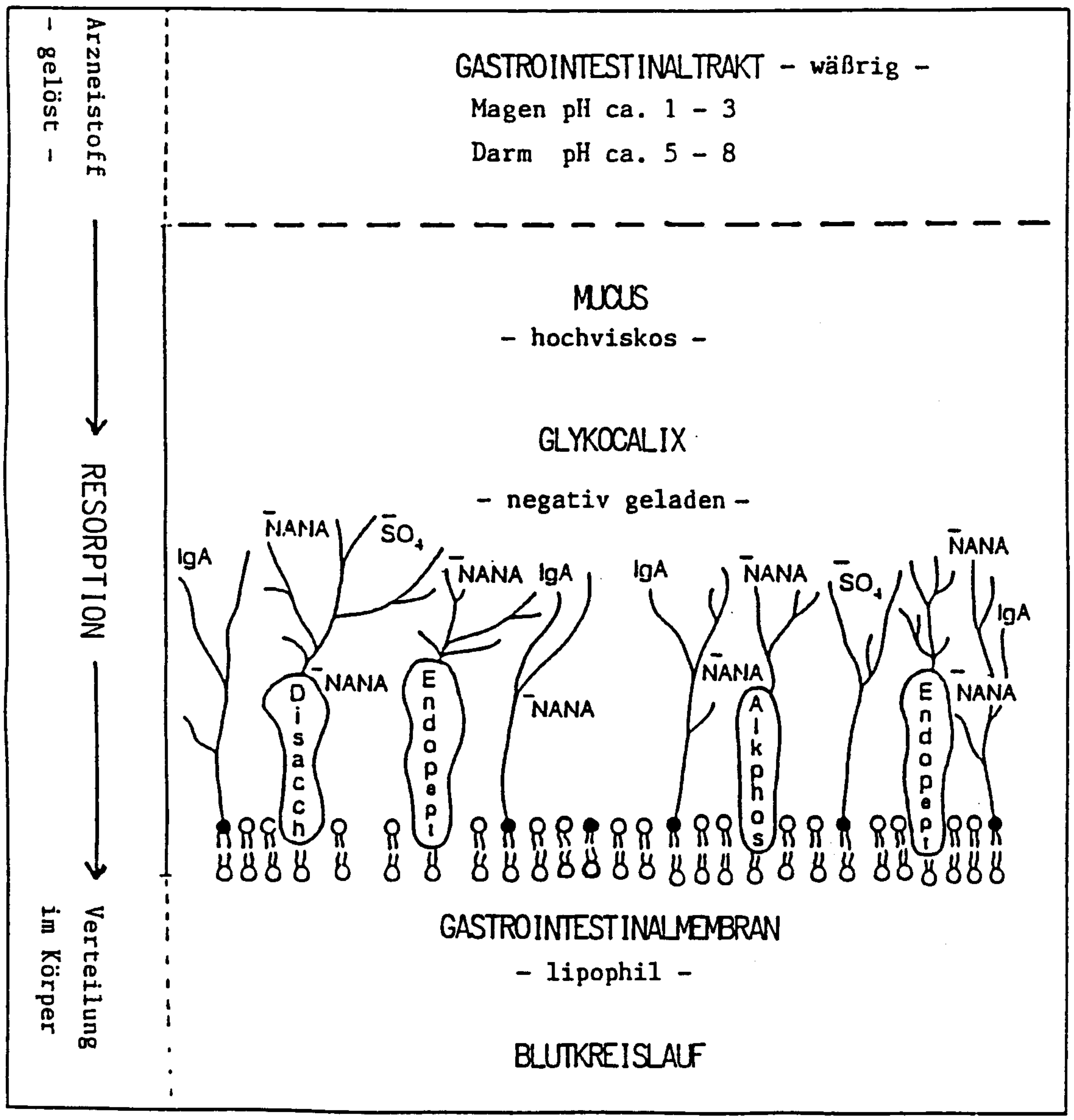
Arzneistoff
- gelöst -
RESORPTION
Verteilung
im Körper
GASTROINTESTINALTRAKT - wäßrig -
Magen pH ca. 1 - 3
Darm pH ca. 5 - 8
MUCUS
- hochviskos -
GLYKOCALIX
- negativ geladen -
IgA
NANA
$SO_4$
Disacch
Endopept
Alkphos
GASTROINTESTINALMEMBRAN
- lipophil -
BLUTKREISLAUF